(19) Europäisches Patentamt / European Patent Office / Office européen des brevets

(11) **EP 4 082 585 B1**

(12) **EUROPÄISCHE PATENTSCHRIFT**

(45) Veröffentlichungstag und Bekanntmachung des Hinweises auf die Patenterteilung:
**04.09.2024   Patentblatt 2024/36**

(21) Anmeldenummer: **21170935.7**

(22) Anmeldetag: **28.04.2021**

(51) Internationale Patentklassifikation (IPC):
**A61L 9/20** (2006.01)        **C02F 1/32** (2023.01)
**C02F 1/00** (2023.01)

(52) Gemeinsame Patentklassifikation (CPC):
**A61L 9/20; C02F 1/325;** A61L 2209/134;
A61L 2209/14; C02F 1/001; C02F 2201/3222;
C02F 2201/328; C02F 2301/043

(54) **REINHALTUNGSVORRICHTUNG**

CLEANING DEVICE

DISPOSITIF DE NETTOYAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.11.2022   Patentblatt 2022/44**

(73) Patentinhaber: **Virobuster International GmbH 53578 Windhagen (DE)**

(72) Erfinder: **FEHMI, Yigit 48599 Gronau (DE)**

(74) Vertreter: **Weidener, Jörg Michael Von Rohr Patentanwälte Partnerschaft mbB Rüttenscheider Straße 62 45130 Essen (DE)**

(56) Entgegenhaltungen:
DE-A1- 3 637 702          DE-U1- 20 2009 018 489
DE-U1- 20 2021 100 433    US-A- 5 933 702
US-A1- 2004 041 564       US-A1- 2009 098 014
US-A1- 2020 368 384

**Beschreibung**

Die Erfindung betrifft eine Reinhaltungsvorrichtung zur Inaktivierung und/oder Abtötung von in einem die Reinhaltungsvorrichtung durchströmenden Medium, insbesondere Wasser oder Luft, nämlich Luft, befindlichen Mikroorganismen, wie Bakterien, Keime, Schimmel und/oder Viren. Die Reinhaltungsvorrichtung weist eine Bestrahlungseinrichtung zur UV-Bestrahlung, insbesondere UVC-Bestrahlung, des Mediums auf.

Insbesondere betrifft die vorliegende Erfindung das technische Gebiet der sogenannten UV-Desinfektion. Der Begriff "UV-Desinfektion" bezeichnet Verfahren, bei denen Mikroorganismen - auch als Mikroben bezeichnet - durch die Behandlung mit UV-Strahlung abgetötet werden können. Dabei kann die UV-Desinfektion zur Trinkwasseraufbereitung, zur Oberflächendesinfizierung, zur Abluftaufbereitung aber auch im Bereich der hygienischen Verarbeitung von Lebensmitteln oder dergleichen eingesetzt werden. Auch Luft in öffentlichen Bereichen kann so "sauber" - das heißt eine geringe Belastung mit schädlichen Mikroorganismen aufweisend - gehalten werden.

Als UV-C-Strahlung wird nachfolgend eine Strahlung mit einer Wellenlänge zwischen 100 nm bis 280 nm verstanden, insbesondere wobei im Rahmen der UV-Desinfektion UV-C-Strahlung bei einer Wellenlänge zwischen 200 nm bis 280 nm, vorzugsweise 240 nm bis 280 nm, eingesetzt wird.

Die UV-Desinfektion nutzt insbesondere eine Wellenlänge der UV-Strahlung zwischen 200 bis 300 nm. Dabei wirkt die abgegebene UV-Strahlung bakterizid - das heißt sie wird von der DNA absorbiert und zerstört deren Struktur und inaktiviert lebende Zellen. So können Mikroorganismen, wie Viren, Bakterien, Hefen und Pilze, mit UV-Strahlung innerhalb kürzester Zeit, insbesondere innerhalb von wenigen Sekunden, unschädlich gemacht werden.

Bei ausreichend hoher Bestrahlungsstärke ist die UV-Desinfektion somit eine zuverlässige und ökologische Methode, da insbesondere keine Zugabe von weiteren Chemikalien notwendig ist. Besonders vorteilhaft ist, dass Mikroorganismen keine Resistenz gegen UV-Strahlung entwickeln können. Letztlich kann durch die UV-Desinfektion auch die Vermehrung der Mikroorganismen unterbrochen werden.

Das Verfahren der UV-Desinfektion kann auch als "Ultraviolet Germicidal Irritation" (UVGI) und/oder als Mikroben-Desinfektion bezeichnet werden, insbesondere wobei UV-Strahlung mit einer Wellenlänge von 254 nm eingesetzt wird. Der Einsatz der UV-Desinfektion ist zur Virusinaktivierung bei der Luftreinigung besonders vorteilhaft, da so ermöglicht wird, große Luftvolumina von Viren zu befreien.

Der Ausbruch der Corona-Pandemie Anfang 2020 zeigt die dringende Notwendigkeit, wirtschaftliche, effiziente und ökologische Verfahren zur Virusinaktivierung vorzuhalten.

Die Luftfiltration auf Basis von sogenannten Hepa-Filtern ist bekannt und fängt insbesondere Partikel wie Viren ab. Auf der anderen Seite gibt es Luftdesinfektionsgeräte, bei denen die Partikel, Keime oder VOC's (volatile organic compounds) nicht abgefangen werden, sondern durch UV-C-Stahlung Ozon, Ionen oder anders behandelt werden.

Der Hauptvorteil der Hepa-Filter ist es, dass sie relativ große Luftmengen bei geringem Energieverbrauch und relativ einfachem mechanischen und technischen Aufbau bewältigen können. Luftdesinfektionsgeräte sind an eine gewisse Expositions- bzw. Behandlungszeit gebunden um eine ausreichende Wirksamkeit zu erreichen. Diese sogenannte Verweilzeit begrenzt die Luftmengenkapazität der Geräte.

Ziel von aktuellen Entwicklungen ist es, Vorrichtungen bereitzustellen, die es ermöglichen, Medienströme mit einem großen Durchsatz, insbesondere Luftströme, effizient reinigen zu können. Ein weiteres oder alternatives Ziel ist es, auch diejenigen Partikel abzutöten, die vergleichsweise resistent gegen UV-Strahlung sind. Letztlich sind verschiedene Grundstrahlungsdosen bekannt, die in Abhängigkeit der zu tötenden Mikroorganismen voneinander abweichen können.

Die UV-Strahlungsdosis ($J/m^2$) ergibt sich aus der Multiplikation der UV-Bestrahlungsintensität ($W/m^2$) und der UV-Bestrahlungszeit (s).

Letztlich hängt die Inaktivierung der Mikroorganismen insbesondere von der Leistung oder der erreichten Intensität der UV-Strahlung ab. Auch die Distanz des Mediumstromes zur Strahlungsquelle hat einen Einfluss auf die Effizienz der Desinfektion bzw. Reinigung.

Problematisch ist in diesem Zusammenhang, dass für eine effiziente Virusinaktivierung der Mikroorganismen eine ausreichend lange Verweildauer in der Bestrahlungseinrichtung bzw. der Bestrahlungskammer sichergestellt werden muss. Gleichzeitig soll auch ein hoher "Durchsatz" des Mediums erreicht werden, so dass insbesondere die von der Reinhaltungsvorrichtung behandelte Gesamtmenge bzw. der insgesamt durch die Reinhaltungsvorrichtung geführte Volumenstrom möglichst hoch sein sollte. Beispielsweise sollen Volumenströme von wenigstens 800 $m^3$/h, bevorzugt wenigstens 1000 $m^3$/h und insbesondere zwischen 1100 bis 2000 $m^3$/h aufbereitet werden. Eine für diesen Volumenstrom konzeptionierte Bestrahlungseinrichtung ist jedoch mit sehr hohen Kosten verbunden - sowohl was den Betrieb als auch die Herstellung anbelangt.

Die US 2020/368384 A1 betrifft ein Luftaufbereitungssystem zur Behandlung von verschmutzter Luft. Das System umfasst einen Luftpartikelfilter sowie mindestens einen Kanal und einen Ventilator, um Luft durch das Luftaufbereitungssystem zu leiten und sie nach der Behandlung auszustoßen, wobei das System ferner eine Luftbehandlungseinheit umfasst, die mindestens eine UV-Lampe aufweist, wobei die Luftbehandlungseinheit stromaufwärts des Luftpartikelfilters angeordnet ist und dazu ausgelegt ist, einen Teilstrom der verschmutzten Luft durch die Luftbehandlungseinheit zu leiten und den Teilstrom einer Photooxidation zu unterziehen.

Die DE 20 2009 018 489 U1 betrifft eine Anlage zur Aufbereitung von Trinkwasser, die eine Partikelfilterstufe und eine erste UV-Desinfektionsstufe sowie eine Trinkwasserentnahmestelle aufweist.

Die US 5 933 702 A betrifft ein Verfahren zur Desinfektion eines Luftstroms, der Mikroorganismen enthält. Dabei wird der Luftstrom mit einem Photokatalysator in Kontakt gebracht.

Die US 2004/041564 A1 betrifft ein Verfahren zur Verbesserung der Raumluftqualität in einer Wohnung, wobei Luft durch ein Luftstromkanal strömt und mindestens ein Teil der Luftströmung mit ultraviolettem Licht bestrahlt wird.

Die DE 20 2021 100 433 U1 betrifft eine Luftfilter-Anordnung zur Reinigung und Desinfektion von Luft. Die Luftfilter-Anordnung weist unter anderem eine UVC-Strahlungsquelle zum Bestrahlen des Luftstroms auf.

Die US 2009/098014 A1 betrifft ein System zur Behandlung von Luft mittels UV-Strahlung.

Die DE 36 37 702 A1 betrifft ein System zur Behandlung von Luft mit UV-Strahlung.

Aufgabe der vorliegenden Erfindung ist es, eine verbesserte Reinhaltungsvorrichtung zur Inaktivierung von in einem die Reinhaltungsvorrichtung durchströmenden Medium befindlichen Mikroorganismen bereitzustellen, insbesondere wobei ein möglichst hoher Durchsatz des die Reinhaltungsvorrichtung durchströmenden Mediums bei gleichzeitiger Verwendung einer Bestrahlungseinrichtung zur UV-Bestrahlung erreicht werden soll.

Die vorgenannte Aufgabe wird erfindungsgemäß durch eine Reinhaltungsvorrichtung zur Inaktivierung bzw. Abtötung von einem die Reinhaltungsvorrichtung durchströmenden Medium, nämlich Luft, befindlichen Mikroorganismen, wie Bakterien, Keime, Schimmel und/oder Viren, mit einer Bestrahlungseinrichtung zur UV-Bestrahlung, insbesondere UV-C-Bestrahlung, des Mediums gemäß Anspruch 1 gelöst. Die Reinhaltungsvorrichtung weist erfindungsgemäß ein mit der Bestrahlungseinrichtung strömungsmäßig verbundenes Leitungsmittel zur Zuführung eines Teilstroms des die Reinhaltungsvorrichtung durchströmenden Stromes des Mediums zu der Bestrahlungseinrichtung auf. Zudem ist insbesondere wenigstens ein weiteres Leitungsmittel zur Führung eines weiteren, insbesondere unbestrahlten, Teilstroms vorgesehen. Das weitere Leitungsmittel kann nicht in der Bestrahlungseinrichtung münden und/oder von der Bestrahlungseinrichtung räumlich und/oder strömungsmäßig getrennt sein. Alternativ oder zusätzlich ist vorgesehen, dass das weitere Leitungsmittel an der Bestrahlungseinrichtung vorbeigeführt ist.

Erfindungsgemäß ist nun die Möglichkeit geschaffen worden, dass bei einer hohen behandelten Luftmenge gleichzeitig auch eine ausreichende Verweilzeit zur Luftdesinfektion ermöglicht werden kann.

Letztlich ist es insbesondere so, dass wenigstens zwei Strömungspfade und/oder Strömungswege vorgesehen sind. Der eine Strömungspfad bzw. Strömungsweg wird durch den in die Bestrahlungseinrichtung geführten Teilstrom gebildet. Dieser Teilstrom wird letztlich in der Bestrahlungseinrichtung mit UV-Strahlung, insbesondere UV-C-Strahlung, behandelt. Zudem wird ein weiterer Strömungspfad bzw. Strömungsweg durch den wenigstens einen weiteren Teilstrom gebildet. Dieser weitere Teilstrom wird nicht in der Bestrahlungseinrichtung behandelt bzw. durch diese geleitet.

Insbesondere ist vorgesehen, dass das weitere Leitungsmittel um die Bestrahlungseinrichtung herumgeführt bzw. daran vorbeigeführt werden kann, insbesondere indem das weitere Leitungsmittel als Bypassleitung zur Bestrahlungsvorrichtung ausgebildet ist. Insbesondere ist das weitere Leitungsmittel strömungsmäßig von dem Leitungsmittel getrennt und/oder räumlich von dem Leitungsmittel getrennt.

Ferner kann der Bestrahlungseinrichtung nur ein (einziger) Teilstrom des die Reinhaltungsvorrichtung durchströmenden Mediumstromes über das Leitungsmittel zuführbar sein, insbesondere indem das Leitungsmittel strömungsmäßig mit der Bestrahlungseinrichtung verbunden ist. Der restliche Strom des die Reinhaltungsvorrichtung durchströmenden Mediums kann insbesondere durch das wenigstens eine weitere Leitungsmittel in wenigstens einem weiteren Teilstrom führbar sein.

Die vorgenannte erfindungsgemäße Strömungsführung ist mit dem Vorteil verbunden, dass der durch die Reinhaltungsvorrichtung insgesamt geführte Gesamtstrom erhöht werden kann, wobei erfindungsgemäß nur ein Teil des Gesamtstromes durch die Bestrahlungseinrichtung geführt werden muss. Beim Zustandekommen der Erfindung ist festgestellt worden, dass überraschenderweise die Behandlung eines Teilstroms des Mediumstromes ausreichend ist, um einen ausreichend hohen Reinheitsgrad - das heißt einen ausreichend hohen Anteil an abgetöteten Mikroorganismen - des gereinigten Mediumstromes zu erreichen. Begründet ist dies erfindungsgemäß darauf, dass durch die erhöhte Gesamtmenge bzw. den erhöhten Gesamtvolumenstrom auch eine verbesserte Effizienz der Inaktivierung erreicht werden kann. Insbesondere wird erfindungsgemäß ein verbesserter Austausch der Luft beispielsweise in geschlossenen Räumen ermöglicht, was mit einer Erhöhung des in der Stunde behandelten Volumenstroms einhergeht. Durch die erfindungsgemäße Bypassleitung bzw. das wenigstens eine weitere Leitungsmittel kann in der Reinhaltungsvorrichtung wenigstens ein (weiterer) Teilstrom bereitgestellt werden, der nicht durch die Bestrahlungseinrichtung behandelt wird, der jedoch vor oder beim Austritt aus der Reinhaltungsvorrichtung mit dem in der Bestrahlungseinrichtung behandelten Volumenstrom gemischt wird. Der so entstehende "Mischstrom" hat erfindungsgemäß weiterhin einen hohen Reinhaltsgehalt bzw. stellt die an eine Luftbehandlungsvorrichtung gestellten Anforderungen sicher. Würde der gesamte höhere Volumenstrom durch die Bestrahlungseinrichtung geführt werden, könnte keine ausreichend lange Verweildauer in der Bestrahlungskammer ermöglicht werden - sofern weiterhin eine unter wirtschaftlichen Gesichtspunkten tragbare Reinhaltungsvorrichtung eingesetzt werden soll.

Die Erfindung ermöglicht somit eine wirtschaftliche und effiziente Reinigung des die Reinhaltungsvorrichtung durchströmenden Mediumstromes.

Die Bestrahlungseinrichtung kann in dem Leitungsmittel angeordnet sein und vorzugsweise durch einen Abschnitt des Leitungsmittels, in dem insbesondere wenigstens eine UV-Strahlungsquelle angeordnet ist, gebildet werden.

Die Innenseite der Bestrahlungseinrichtung und/oder des Leitungsmittels im Bereich der Bestrahlungseinrichtung kann insbesondere zumindest bereichsweise, vorzugsweise vollflächig, reflektierend ausgebildet sein und insbesondere einen Reflektionsgrad für die UV-Strahlung von wenigstens 0,6 aufweisen.

In der Bestrahlungseinrichtung ist erfindungsgemäß eine Strahlungsquelle zur Bestrahlung des die Bestrahlungseinrichtung durchströmenden Mediums bzw. des Teilstroms vorgesehen.

Das Innere der Bestrahlungseinrichtung bzw. der umschlossene Innenraum der Bestrahlungseinrichtung und/oder des Leitungsmittels kann insbesondere als UV-Behandlungskammer zur Behandlung des Teilstroms bzw. des in durch die Bestrahlungseinrichtung geführten Teilstroms des Mediums angesehen werden. Bevorzugt ist die Innenwandung ein Reflektor, der weiter bevorzugt als eingeschobene und/oder austauschbare Gehäusekomponente ausgebildet ist.

Unter dem Reflektionsgrad wird im Sinne der vorliegenden Erfindung das Verhältnis zwischen reflektierter und einfallender Intensität als Energiegröße verstanden. Der Reflektionsgrad kann insbesondere in Abhängigkeit des Materials der Innenwandung, auf die die Strahlung auftrifft, von der Strahlung selbst abhängen.

Erfindungsgemäß gelingt es insbesondere, auch Bakterien, Viren und/oder Pilze mit einer vergleichsweise hohen UV-Resistenz abzutöten, insbesondere indem eine möglichst lange Verweildauer in der Bestrahlungseinrichtung sichergestellt werden kann.

Durch die Erhöhung der Intensität der UV-Strahlung kann somit die Strahlungsdosis erhöht werden, insbesondere bei der gleichen Zeitdauer der Aussetzung der Mikroorganismen der UV-C-Strahlung.

Zudem wird die Effizienz der UV-Desinfektion beeinflusst durch den Widerstandswert der Mikroorganismen (k [m²/J]) gegen die UV-Strahlung, die Aussetzungszeit (t [s]) sowie durch die Intensität der Strahlung (I [W/m²]). Der Widerstandswert k der Mikroorganismen kann nicht beeinflusst werden und kann insbesondere zwischen 0,001 bis 0,5 m²/J, bevorzugt zwischen 0,0014 bis 0,3 m²/J liegen.

Erfindungsgemäß kann erreicht werden, dass bei einer vorgegebenen UV-Resistenz (k-Faktor) eine geringe Aussetzungszeit durch die Erhöhung der Gesamt-Intensität ausreichend ist, da die Größen sich letztlich insbesondere gegenseitig beeinflussen. So kann sowohl durch eine Erhöhung der Aussetzungszeit als auch durch eine Erhöhung der Intensität die Inaktivierung der Mikroorganismen nach der Überwindung der UV-Resistenz erreicht werden.

Die Abtötungsrate der Mikroorganismen kann insbesondere nach folgender Formel bestimmt werden:

$$S\,(t) = S_o\,e^{-I\,\cdot\,k\,\cdot\,t}$$

mit:

I =    Intensität (W/m²),
k =    UV-Rate des Mikroorganismus (m²/J),
t =    die Verweilzeit (s)
S =    Mikrobengehalt
$S_o$ =    Anfangsgehalt der Mikroben.

Insbesondere kann mit der erfindungsgemäßen Reinhaltungsvorrichtung wenigstens eine sechs-fache ACH (air change per hour), die beispielsweise in Klassenräumen benötigt wird, erfüllt werden, insbesondere mit einem zu behandelnden Volumenstrom von circa 1200 m³ pro Stunde.

Erfindungsgemäß kann die Reinhaltungsvorrichtung ein mobiles oder an wenigstens einer Wand oder einer Decke montierbares Luftdesinfektionsgerät sein. Es kann sich aber auch um eine stationäre Anlage handeln.

Für die Auslegung der Reinhaltungsvorrichtung ist die reale Abscheide-Behandlungsmenge der behandelten Mikroorganismen entscheidend, die erfindungsgemäß bei einem hohen behandelten Gesamtvolumenstrom die gesetzlichen Anforderungen erfüllen kann. Diese reale Abscheide-Behandlungs-Volumenleistung wird als CADR (clean air delivery rate) bezeichnet und stellt letztlich eine Vergleichsmöglichkeit dar. Für den CADR sind Haupteinflussfaktoren beispielsweise der behandelte Volumenstrom Q durch die Reinhaltungsvorrichtung, der interne Abscheide-Behandlungswirkungsgrad N beim Durchströmen des Luftstroms und der sogenannte Fluidal Efficiency Factor (dimensionslos F) des gerätezeugten Luftstroms in einem Raum. Der CADR kann also durch folgende Funktionen beschrieben werden:

$$CADR = Q\,[m^3/h] \bullet N\,[\%] \bullet F$$

Wenn der geforderte CADR bekannt ist, kann somit die behandelte Luftmenge abgeleitet werden. Die Bestimmung des Kennwertes F spielt somit aus konstruktionstechnischen Gesichtspunkten eine große Rolle.

Erfindungsgemäß kann insbesondere eine hohe Behandlungsdosis des in der Bestrahlungseinrichtung behandelten Teilstroms ermöglicht werden.

Die ist beispielsweise zur effizienten Abtötung des Corona-Viruses notwendig.

Letztlich kann erfindungsgemäß eine Lösung bereitgestellt werden, bei der eine hohe Reinigungsleistung durch die Aufteilung der Ströme erreicht wird, die unerwarteter Weise deutlich besser ist, als wäre der Gesamtstrom durch die Bestrahluntseinrichtung geführt worden.

Bei einer besonders bevorzugten Ausführungsform ist vorgesehen, dass die Reinhaltungsvorrichtung ein einen Einlass und einen Auslass für das Medium aufweisendes Gehäuse aufweist. Insbesondere ist das Gehäuse von dem durch die Reinhaltungsvorrichtung geführten Strom des Mediums durchströmbar. Alternativ oder zusätzlich kann vorgesehen sein, dass die Bestrahlungseinrichtung in dem Gehäuse bzw. in dem Inneren des Gehäuses angeordnet ist.

Vorzugsweise verbindet das Leitungsmittel den Einlass mit der Bestrahlungseinrichtung strömungsmäßig. Alternativ oder zusätzlich kann vorzugsweise das wenigstens eine weitere Leitungsmittel strömungsmäßig mit dem Einlass verbunden sein.

Insbesondere ist das weitere Leitungsmittel strömungsmäßig nicht mit der Bestrahlungseinrichtung, aber mit dem Einlass verbunden. Das weitere Leitungsmittel kann dabei von dem Leitungsmittel abgehen oder mit dem Leitungsmittel zusammengeführt werden - und zwar nachdem der Teilstrom die Bestrahlungseinrichtung passiert hat. So kann das Leitungsmittel auch in Strömungsrichtung hinter der Bestrahlungseinrichtung weitergeführt werden und vorzugsweise auch mit dem Auslass verbunden sein.

Insbesondere können demnach das weitere Leitungsmittel und/oder das Leitungsmittel strömungsmäßig den Einlass mit dem Auslass verbinden.

Bei einer besonders bevorzugten Ausführungsform ist vorgesehen, dass eine Mehrzahl von weiteren Leitungsmitteln zur Führung einer Mehrzahl von weiteren Teilströmen vorgesehen ist. Insbesondere sind in der erfindungsgemäßen Reinhaltungsvorrichtung wenigstens 3, bevorzugt zwischen 3 bis 10, weiter bevorzugt zwischen 4 bis 5, weitere Leitungsmittel angeordnet. Die weiteren Leitungsmittel können insbesondere die Bestrahlungseinrichtung umgeben bzw. um die Bestrahlungseinrichtung herum bzw. neben dieser angeordnet sein. In diesem Zusammenhang versteht es sich, dass die weiteren Leitungsmittel derart ausgebildet sind, dass sie nicht in die Bestrahlungseinrichtung münden und/oder an der Bestrahlungseinrichtung vorbeigeführt sind und/oder von der Bestrahlungseinrichtung räumlich und/oder strömungsmäßig getrennt sind. Insbesondere sind die weiteren Leitungsmittel als Bypassleitungen zur Bestrahlungseinrichtung ausgebildet. Der durch die weiteren Leitungsmittel geführte Teilstrom hat demgemäß einen Strömungspfad bzw. einen Strömungsweg, der nicht in oder durch die Bestrahlungseinrichtung führt, sondern von der Bestrahlungseinrichtung (strömungsmäßig) getrennt ist.

Vorzugsweise ist das Leitungsmittel und/oder das wenigstens weitere Leitungsmittel, bevorzugt alle weiteren Leitungsmittel, als, bevorzugt geschlossener, Strömung-Führungskanal ausgebildet. Der Strömung-Führungskanal kann insbesondere ein Rohr sein. Letztlich dient das Leitungsmittel und das weitere Leitungsmittel dazu, die jeweilige Teilströmung zu führen und den diesbezüglichen Strömungsweg des jeweiligen Teilstromes vorzugeben.

Insbesondere kann der Einlass eine Mehrzahl von Einlassöffnungen, bevorzugt zum Ansaugen von Mediumstrom, aufweisen. Alternativ oder zusätzlich kann vorgesehen sein, dass der Auslass eine Mehrzahl von Auslassöffnungen aufweist. Demnach muss der Einlass und/oder der Auslass nicht zwingend durch eine einzige Öffnung gebildet werden, sondern der gesamte Bereich, der zum Ansaugen (Einlass) bzw. Abgeben (Auslass) des Mediums vorgesehen ist, dient als Einlass bzw. Auslass.

Besonders bevorzugt ist in einer weiteren Ausführungsform vorgesehen, dass dem Leitungsmittel wenigstens eine Einlassöffnung und/oder dem wenigstens einen weiteren Leitungsmittel wenigstens eine weitere Einlassöffnung zugeordnet ist. Insbesondere kann die dem Leitungsmittel zugeordnete wenigstens eine Einlassöffnung nicht gleichzeitig dem weiteren Leitungsmittel zugeordnet sein. Somit kann der durch das Leitungsmittel und durch das weitere Leitungsmittel geführte jeweilige Teilstrom über unterschiedliche Einlassöffnungen der Reinhaltungsvorrichtung zugeführt werden.

Darüber hinaus kann dem Leitungsmittel wenigstens eine Auslassöffnung und/oder dem wenigstens einen weiteren Leitungsmittel wenigstens eine weitere Auslassöffnung zugeordnet sein. Insbesondere ist die Auslassöffnung für das Leitungsmittel nicht dem weiteren Leitungsmittel zugeordnet. So können in Abhängigkeit der Leitungsmittel unterschiedliche Auslassöffnungen bereitgestellt werden. In diesem Zusammenhang versteht es sich, dass dem Leitungsmittel oder dem weiteren Leitungsmittel auch eine Mehrzahl von Auslassöffnungen zum Abgeben des Mediumstromes zugeordnet sein können. Die Auslassöffnungen oder die Einlassöffnungen können zueinander benachbart oder zueinander beabstandet angeordnet und insbesondere als Schlitze ausgebildet sein.

Vorzugsweise ist ein strömungsmäßig mit dem Einlass verbundenes Einlassmittel, insbesondere ein Einlasskanal und/oder Einlassrohr, vorgesehen. Das Einlassmittel kann in das Leitungsmittel und das wenigstens eine weitere Leitungsmittel münden. Insbesondere kann das Einlassmittel in das Leitungsmittel und das wenigstens eine weitere Leitungsmittel aufgezweigt werden. In den diesbezüglichen Aufzweigungen können zur Steuerung des Volumenstromes Ventile angeordnet sein. Somit können sich das Leitungsmittel und das weitere Leitungsmittel von dem Einlassmittel abzweigen, bedarfsweise entsprechend gesteuert. Das Einlassmittel kann mit den Einlassöffnungen strömungsmäßig verbunden sein bzw. die Einlassöffnungen können in das Einlassmittel übergehen.

Ferner ist bei einer weiteren bevorzugten Ausführungsform vorgesehen, dass ein strömungsmäßig mit dem Auslass verbundenes Auslassmittel vorgesehen ist. Das Auslassmittel kann insbesondere in die Auslassöffnungen des Auslasses münden. Das Auslassmittel kann letztlich als gemeinsamer Kanal bzw. gemeinsames Rohr für den Mediumstrom ausgebildet sein. Das Leitungsmittel und/oder der aus der Bestrahlungseinrichtung austretende Teilstrom können in das Auslassmittel münden. Zudem kann auch das wenigstens eine weitere Leitungsmittel in das Auslassmittel münden. Insbesondere können in dem Auslassmittel die einzelnen Teilströme zusammengeführt und vorzugsweise miteinander vermischt werden und so letztlich gemeinsam aus der Reinhaltungsvorrichtung austreten. Durch die Mischung der Teilströme wird insbesondere erreicht, dass ein aus der Reinhaltungsvorrichtung austretender Gesamtmediumstrom mit einer hohen Effizienz der Luftreinhaltung bereitgestellt werden kann.

Erfindungsgemäß ist die Reinhaltungsvorrichtung derart ausgebildet, dass der aus der Bestrahlungseinrichtung austretende Teilstrom eine höhere Geschwindigkeit als der wenigstens eine weitere Teilstrom aufweist, der in dem wenigstens einen weiteren Leitungsmittel geführt wird bzw. dessen Strömungsweg entlang des weiteren Leitungsmittels gerichtet ist. Insbesondere kann der aus der Bestrahlungseinrichtung austretende Teilstrom eine um wenigstens 20 %, bevorzugt wenigstens 30 %, weiter bevorzugt wenigstens 40 %, und/oder um wenigstens 1 m/s, bevorzugt um wenigstens 2 m/s, höhere Geschwindigkeit als der wenigstens eine weitere Teilstrom aufweisen. Die höhere Geschwindigkeit geht mit dem vorteilhaften strömungstechnischen Effekt einher, dass der aus der Bestrahlungseinrichtung austretende Teilstrom insbesondere dazu eingerichtet ist, den wenigstens einen weiteren Teilstrom "mitzuziehen" und so letztlich eine sogenannte "Venturiströmung" bereitzustellen (venturi drag flow). Insbesondere ermöglicht die höhere Geschwindigkeit, dass zur Erzeugung des weiteren Teilstroms an sich keine eigenen Gebläseeinrichtungen oder dergleichen benötigt werden. Die Strömung des weiteren Teilstroms kann letztlich durch die Zugwirkung des die höhere Geschwindigkeit aufweisenden und aus der Bestrahlungseinrichtung austretenden Teilstroms sichergestellt werden.

Bevorzugt ist in dem wenigstens einen weiteren Leitungsmittel, vorzugsweise in jedem weiteren Leitungsmittel, eine Filtereinrichtung angeordnet. Die Filtereinrichtung kann insbesondere als sogenannter Hepa-Filter ausgebildet sein. Alternativ oder zusätzlich kann vorgesehen sein, dass die Filtereinrichtung derart ausgebildet ist, dass Partikel mit einem Durchmesser von größer 1 $\mu$m, bevorzugt größer 0,5 $\mu$m, aus dem Mediumstrom gefiltert werden. Der Hepa-Filter wird auch als Schwebstofffilter bezeichnet, der zur Abscheidung von Schwebstoffen aus der Luft ausgebildet sein kann. In der Filtereinrichtung können Filtermatten eingesetzt werden. Insbesondere darf auf die europäische Norm zur Klassifizierung der Schwebstofffilter verwiesen werden, nämlich die EN 1822-1:2009 (Stand April 2021).

Darüber hinaus kann auch vor dem Einlass und/oder in dem Leitungsmittel, bevorzugt in Strömungsrichtung vor der Bestrahlungseinrichtung bzw. dem die Bestrahlungseinrichtung bildenden Abschnitt des Leitungsmittels, ein Vorfilter angeordnet sein. Insbesondere ist der Vorfilter als Schwebstofffilter bzw. Hepa-Filter ausgebildet. Alternativ oder zusätzlich kann vorgesehen sein, dass der Vorfilter derart ausgebildet ist, dass Partikel mit einem Durchmesser von größer als 1 $\mu$m, bevorzugt größer als 0,5 $\mu$m, aus dem Mediumstrom gefiltert werden.

Somit können insbesondere aus dem Mediumstrom solche Partikel herausgefiltert werden, die andernfalls eine sogenannte "Schattenbildung" in der UV-Behandlungskammer bei der entstehenden Wechselwirkung mit der UV-Strahlung erzeugen würden. Letztlich ist in diesem Zusammenhang relevant, dass die UV-Strahlung in einer Größenordnung zwischen 0,2 bis 0,3 $\mu$m liegt. Da die vorgenannten Partikel, beispielsweise Staubpartikel oder Pollen oder dergleichen, jedoch einen größeren Durchmesser als die Wellenlänge aufweisen, kann die Wellenlänge insbesondere nicht die Partikel mit einem Durchmesser von größer als 1 $\mu$m passieren. Ein Bakterium kann beispielsweise einen Durchmesser von circa 0,3 $\mu$m aufweisen. Erfindungsgemäß ist festgestellt worden, dass der Schatteneffekt vor Partikeln mit einem Durchmesser unter 0,5 $\mu$m, insbesondere zwischen 0,3 $\mu$m bis 0,5 $\mu$m, zwar vorhanden ist, jedoch noch die erreichte Abtötung der Mikroorganismen toleriert werden kann. So können auch diejenigen Mikroorganismen mit einem Durchmesser größer als die Wellenlänge der UV-Strahlung ebenfalls unschädlich gemacht werden, da auch auf sie die UV-Strahlung trifft.

Eine Vorfilterung ermöglicht den Vorteil, dass insbesondere der Bestrahlungseinrichtung ein solcher Teilstrom zugeführt werden kann, der bereits einer Filterstufe unterlegen hat. Somit kann in der Bestrahlungseinrichtung eine effiziente Desinfektion des Teilstromes erfolgen.

In weiteren Ausführungsbeispielen kann auch vorgesehen sein, dass in wenigstens einem weiteren Leitungsmittel ebenfalls eine weitere Bestrahlungseinrichtung zur UV-Bestrahlung, insbesondere zur UV-C-Bestrahlung, des weiteren Teilstroms angeordnet ist. Durch die weitere Bestrahlungseinrichtung, die insbesondere eine weitere Strahlungsquelle zum Abgeben der UV-Strahlung aufweist, kann die Effizienz bzw. der gesamt erreichte Abscheidegrad der in dem Mediumstrom befindlichen Mikroorganismen erhöht werden. Letztlich kann die Reinigung des in dem weiteren Leitungsmittel geführten Teilstroms insbesondere zumindest im Wesentlichen unabhängig von der Reinigung des in dem Leitungsmittel geführten Teilstroms ausgebildet werden. Eine Anpassung an unterschiedliche Benutzungssituationen und/oder Vorgaben kann demgemäß erfolgen. So kann auch vorgesehen sein, dass in der Reinhaltungsvorrichtung beispielsweise zwei Bestrahlungseinrichtungen vorgesehen sind, wobei eine Bestrahlungseinrichtung strömungsmäßig mit dem Leitungsmittel und eine weitere Bestrahlungseinrichtung strömungsmäßig mit dem weiteren Leitungsmittel verbunden ist. Zudem können weitere Leitungsmittel vorgesehen sein, in denen keine Bestrahlungseinrichtung angeordnet ist. Eine solche Anordnung ist beispielsweise zur Behandlung eines hohen Volumenstromes besonders vorteilhaft.

Die weitere Bestrahlungseinrichtung kann zudem durch einen Abschnitt des weiteren Leitungsmittels gebildet werden, in dem die weitere Strahlungsquelle angeordnet ist. Für weitere Ausführungen zu der weiteren Bestrahlungseinrichtung darf auf die Ausführung zur Bestrahlungseinrichtung verwiesen werden, die in gleicher Weise für die weitere Bestrahlungseinrichtung gelten.

Bei einer besonders bevorzugten Ausgestaltung des Erfindungsgedankens ist vorgesehen, erfindungsgemäß ist vorgesehen, dass eine Gebläseeinrichtung zum Ansaugen und/oder Abblasen des Mediums, insbesondere zum Erzeugen des Mediumstromes vom Einlass zum Auslass, vorgesehen ist. Die Gebläseeinrichtung kann dem Leitungsmittel und/oder dem weiteren Leitungsmittel zugeordnet sein. Letztlich stellt die Gebläseeinrichtung sicher, dass das Medium über den Einlass die Reinhaltungsvorrichtung durchströmen kann. Die Gebläseeinrichtung kann dabei im Hinblick auf den Strömungsweg des die Reinhaltungsvorrichtung durchströmenden Mediums an unterschiedlichen Stellen angeordnet sein. So kann die Gebläseeinrichtung entweder dem Einlass oder dem Auslass zugeordnet sein. Besonders bevorzugt ist, dass die Gebläseeinrichtung im Bereich des Einlasses bzw. im Hinblick auf den Strömungsweg des Teilstroms vor der Bestrahlungseinrichtung angeordnet ist. Grundsätzlich ist aber auch in weiteren Ausführungsformen möglich, dass die Gebläseeinrichtung im Hinblick auf den Strömungspfad des Teilstroms hinter der Bestrahlungseinrichtung angeordnet ist. Die Anordnung der Gebläseeinrichtung kann insbesondere in Abhängigkeit von unterschiedlichen Kundenbedürfnissen ausgewählt werden.

Vorzugsweise weist die Gebläseeinrichtung einen Ventilator zum Erzeugen des Teilstroms in dem Leitungsmittel von dem Einlass zu der Bestrahlungseinrichtung, bevorzugt zu dem Auslass, auf. Insbesondere kann die Gebläseeinrichtung auch eine Mehrzahl von Ventilatoren aufweisen. So kann die Gebläseeinrichtung auch wenigstens einen weiteren Ventilator aufweisen, der zum Erzeugen des wenigstens einen weiteren Teilstroms ausgebildet ist, nämlich zum Erzeugen des Teilstroms in dem wenigstens einen weiteren Leitungsmittel von dem Einlass zu dem Auslass. Die Anzahl der Ventilatoren der Gebläseeinrichtung kann in Abhängigkeit des Volumenstroms des die Reinhaltungsvorrichtung durchströmenden Mediums ausgewählt werden.

Erfindungsgemäß weist die Gebläseeinrichtung ein Stellmittel zur Steuerung des Volumenstroms des in das Leitungsmittel geführten Teilstroms auf. Erfindungsgemäß weist die Gebläseeinrichtung wenigstens ein weiteres Stellmittel zur Steuerung des Volumenstroms des wenigstens einen in das wenigstens eine weitere Leitungsmittel geführten weiteren Teilstroms auf. Als Stellmittel und/oder weiteres Stellmittel kann ein Ventil und/oder eine Stellklappe vorgesehen sein. So ermöglicht das Stellmittel in Kombination mit dem wenigstens einen weiteren Stellmittel, dass die Volumenströme der jeweiligen Teilströme in Abhängigkeit von unterschiedlichen Vorgaben individuell gesteuert werden können. So kann beispielsweise der der Bestrahlungseinrichtung zugeführte Teilstrom im Hinblick auf den Volumenstrom angepasst werden, insbesondere so dass eine ausreichende Verweilzeit in der Bestrahlungseinrichtung sichergestellt werden kann. Auch können die weiteren Stellmittel dazu ausgebildet sein, dass bei einer Mehrzahl von weiteren Leitungsmitteln eine Zuführung der jeweiligen weiteren Teilströme zu den jeweiligen weiteren Leitungsmitteln gelingt. Letztlich ermöglichen die Stellmittel, dass eine Auftrennung des Mediumstromes in einzelne Teilströme erfolgt.

Die Bestrahlungsdosis beträgt insbesondere im Rahmen der vorliegenden wenigstens 30 J/m$^2$, bevorzugt wenigstens 50 J/m$^2$, weiter bevorzugt wenigstens 70 J/m$^2$ und vorzugsweise 100 J/m$^2$ +/- 20 %.

Insbesondere kann die Bestrahlungseinrichtung zur Behandlung eines Volumenstroms des Teilstroms von wenigstens 100 m$^3$/h, bevorzugt von wenigstens 200 m$^3$/h, weiter bevorzugt zwischen 300 bis 600 m$^3$/h, ausgebildet sein. Bei den vorgenannten Volumenströmen wird eine ausreichende Verweilzeit mit einer ausreichend hohen Bestrahlungsdosis zur effizienten Abtötung der in dem Mediumstrom befindlichen Mikroorganismen durch die Bestrahlungseinrichtung sichergestellt. Der gesamte Volumenstrom kann jedoch höher als der durch die Bestrahlungseinrichtung geführte Teilstrom sein.

In diesem Zusammenhang ist es besonders bevorzugt, dass die Reinhaltungsvorrichtung derart ausgebildet ist, dass der Volumenstrom des durch das Leitungsmittel geführten Teilstroms an den Gesamtvolumenstrom des die Reinhaltungsvorrichtung durchströmenden Mediumstroms ein Anteil von wenigstens 10 %, bevorzugt zwischen 20 % bis 80 %, weiter bevorzugt zwischen 30 % bis 50 %, aufweist. Die vorgenannten Angaben verdeutlichen, dass der gesamte Volumenstrom höher als der Volumenstrom des durch die Bestrahlungseinrichtung geführten Teilstromes sein kann. Insbesondere kann in der Reinhaltungsvorrichtung ein Volumenstrom von wenigstens 800 m$^3$/h, bevorzugt von wenigstens 1000 m$^3$/h, weiter bevorzugt zwischen 1000 bis 3000 m$^3$/h, behandelt werden.

Besonders bevorzugt ist, wenn das weitere Leitungsmittel zumindest im Wesentlichen parallel zu dem Leitungsmittel angeordnet ist. Wie zuvor erläutert, kann das Leitungsmittel von den weiteren Leitungsmitteln umgeben sein. Auch die Bestrahlungseinrichtung kann bevorzugt von den weiteren Leitungsmitteln umgeben sein. In diesem Zusammenhang versteht es sich auch, dass die Auslassöffnung, in die das weitere Leitungsmittel mündet, an einer anderen Wandung angeordnet sein kann als die Auslassöffnung, in die das Leitungsmittel mündet. Bevorzugt ist jedoch, wenn die Auslassöffnung für das Leitungsmittel und das weitere Leitungsmittel an derselben Wandung des Gehäuses und insbesondere zueinander benachbart angeordnet sind. Insbesondere ist es bevorzugt, wenn das Leitungsmittel und das wenigstens eine weitere Leitungsmittel in derselben Auslassöffnung mündet.

Erfindungsgemäß ist vorgesehen, dass eine Steuerungseinrichtung zur Steuerung, Zuschaltung, Abschaltung und/oder Regelung des Volumenstroms des Teilstroms und/oder des wenigstens einen weiteren Teilstroms vorgesehen ist. Insbesondere ist die Steuerungseinrichtung zur Steuerung und/oder Regelung des Stellmittels und/oder des wenigstens einen weiteren Stellmittels und/oder der Gebläseeinrichtung ausgebildet. Durch die Steuerungseinrichtung kann insbesondere der Gesamtvolumenstrom, der durch die Reinhaltungsvorrichtung geführt wird, verändert und/oder angepasst werden. Gleichzeitig kann durch die Steuerungseinrichtung auch der durch die Bestrahlungseinrichtung geführte Teilstrom angepasst werden.

Ferner ist es besonders bevorzugt, dass das Leitungsmittel und/oder das weitere Leitungsmittel und/oder das weitere Leitungsmittel zumindest im Wesentlichen vollständig in dem Gehäuse angeordnet ist.

Vorzugsweise weist das Gehäuse und/oder die Reinhaltungsvorrichtung eine Länge zwischen 30 bis 200 cm, bevorzugt zwischen 80 bis 150 cm, auf. Das Gehäuse und/oder die Reinhaltungsvorrichtung kann einen Durchmesser, vorzugsweise einen Innendurchmesser, zwischen 8 bis 30 cm, bevorzugt zwischen 10 bis 20 cm, aufweisen. Bei einer besonders bevorzugten Ausführungsform weist das Gehäuse und/oder die Reinhaltungsvorrichtung eine Länge zwischen 80 bis 150 cm und einen Innendurchmesser zwischen 10 bis 20 cm auf. Bei den vorgenannten Größenordnungen kann insbesondere für einen Mediumstrom mit einer Strömungsgeschwindigkeit zwischen 1 bis 2 m/s eine effiziente Inaktivierung der Mikroorganismen gewährleistet werden.

Darüber hinaus kann die erfindungsgemäße Reinhaltungsvorrichtung auch in Klimaanlagen oder Bürogebäuden oder dergleichen eingesetzt werden. Dabei versteht es sich, dass die Reinhaltungsvorrichtung in Abhängigkeit des Mediumstromes und/oder des Durchsatzes des Mediumstromes ausgebildet sein kann.

Ferner kann bei einer weiteren bevorzugten Ausführungsform vorgesehen sein, dass die Strahlungsquelle eine Mehrzahl von Leuchtmitteln, vorzugsweise LEDs, aufweist. Darüber hinaus kann alternativ oder zusätzlich die Strahlungsquelle eine zumindest im Wesentlichen langgestreckte und/oder stabförmige Form aufweisen. Insbesondere kann die Strahlungsquelle eine Länge von wenigstens 5 cm, bevorzugt zwischen 5 cm bis 30 cm, weiter bevorzugt zwischen 10 cm bis 20 cm, aufweisen. Bei einer besonders bevorzugten Ausführungsform ist vorgesehen, dass die Strahlungsquelle eine langgestreckte Form mit einer Mehrzahl von Leuchtmitteln, vorzugsweise LEDs aufweist, die entlang eines "arrays" angeordnet sind.

Zudem kann die Strahlungsquelle einen Durchmesser von wenigstens 1 cm, bevorzugt zwischen 1 cm und 20 cm, weiter bevorzugt zwischen 2 cm und 10 cm und insbesondere von 5 cm +/- 1 cm, aufweisen. Der Durchmesser der Strahlungsquelle kann insbesondere auch von dem Durchmesser des in der Strahlungsquelle eingesetzten Leuchtmittels abhängen.

Alternativ oder zusätzlich kann die Strahlungsquelle auch als UV-Niederdrucklampen, insbesondere eine Niederdruck-Quecksilber-Entladungslampe, und/oder als UV-Mitteldrucklampe ausgebildet sein.

Letztlich kann die Strahlungsquelle die zur Inaktivierung der Mikroorganismen benötigte UV-Strahlung, insbesondere die UV-C-Strahlung, bereitstellen. Dabei kann die Strahlungsquelle an ihrer Oberfläche eine Intensität zwischen 1000 bis 8000 W/m$^2$, bevorzugt zwischen 2000 bis 6000 W/m$^2$ und insbesondere von 4200 W/m$^2$ +/- 20 %, aufweisen.

Des Weiteren kann die Strahlungsquelle insbesondere eine Leistung von wenigstens 100 W, bevorzugt von 190 W +/- 10 %, erbringen. Die Leistung im Bereich der UV-C-Strahlung kann bevorzugt zwischen 10 bis 100 W, weiter bevorzugt zwischen 50 bis 70 W, betragen. Die von der Strahlungsquelle emittierte Strahlung kann mit ihrer Intensität im Abstandsquadrat abnehmen.

Bei einer weiteren bevorzugten Ausführungsform des Erfindungsgedankens ist vorgesehen, dass eine Mehrzahl von Strahlungsquellen in der Bestrahlungseinrichtung und/oder den die Bestrahlungseinrichtung bildenden Abschnitt des Leitungsmittels angeordnet ist. Insbesondere können in der Bestrahlungseinrichtung und/oder den die Bestrahlungseinrichtung bildenden Abschnitt des Leitungsmittels zwischen 2 bis 10, bevorzugt zwischen 2 bis 5, Strahlungsquellen angeordnet sein. Die Strahlungsquellen können jeweils insbesondere ebenfalls eine Mehrzahl von Leuchtmitteln, vorzugsweise LEDs, aufweisen. Durch die mehreren Strahlungsquellen kann über die Länge der Bestrahlungseinrichtung eine zumindest im Wesentlichen gleichmäßige und/oder zur Inaktivierung der Mikroorganismen ausreichende Strahlung bereitgestellt werden.

Die Strahlung der mehreren Strahlungsquellen kann des Weiteren auch in dem entstehenden Interferenzbild in der UV-Behandlungskammer miteinander wechselwirken und zur Erhöhung der konstruktiven Interferenz beitragen. Die erfindungsgemäß angegebene Eignung zur konstruktiven Interferenz gilt insbesondere für jede Strahlungsquelle, die in der erfindungsgemäßen Bestrahlungseinrichtung eingesetzt wird.

Grundsätzlich ist es auch möglich, dass die Strahlungsquellen voneinander unterschiedlich ausgebildet sind. Bevorzugt wird eine zumindest im Wesentlichen baugleiche Ausbildung der Strahlungsquellen vorgesehen.

Der Mediumstrom bzw. der jeweilige Teilstrom kann zumindest im Wesentlichen laminar durch das Leitungsmittel und/oder das weitere Leitungsmittel geführt werden. Alternativ oder zusätzlich kann aber auch vorgesehen sein, dass der durchströmende jeweilige Teilstrom eine turbulente Strömung ausbildet.

Erfindungsgemäß emittiert mindestens eine Strahlungsquelle UV-Strahlung in einem Wellenlängenbereich von 250 bis 285 nm, weiter bevorzugt von 270 bis 280 nm und insbesondere von 254 nm +/- 10 % und/oder von 278 nm +/- 10 %. Bei einer UV-C-Strahlung mit einer Wellenlänge von 254 nm +/- 10 % kann insbesondere eine hohe Vireninaktivierung erreicht werden. UV-Strahlung mit einer Wellenlänge in der vorgenannten Größenordnung ermöglicht erfindungsgemäß die Abtötung der Mikroorganismen in dem Mediumstrom.

Darüber hinaus betrifft die vorliegende Erfindung ein Verfahren zur Inaktivierung von in einem eine Reinhaltungsvorrichtung der vorgenannten Art durchströmenden Medium befindliche Mikroorganismen. Als Medium ist insbesondere Wasser oder Luft vorgesehen. Die Mikroorganismen in dem Medium können auch abgetötet werden. Als Mikroorganismen können Bakterien, Keime, Schimmel und/oder Viren vorgesehen sein. Bei dem erfindungsgemäßen Verfahren durchströmt das Medium die Reinhaltungsvorrichtung. Der Bestrahlungseinrichtung wird nur ein Teilstrom des die Reinhaltungsvorrichtung insgesamt durchströmenden Gesamtstromes des Mediums über das Leitungsmittel zugeführt. Der Strömungsweg des Teilstroms beginnt insbesondere beim Einlass und endet in der Bestrahlungseinrichtung und/oder dem Auslass. Wenigstens ein weiterer Teilstrom wird durch das weitere Leitungsmittel geführt. Der weitere Teilstrom ist insbesondere strömungsmäßig getrennt von dem Teilstrom und/oder wird nicht durch die Bestrahlungseinrichtung geführt. Letztlich kann der weitere Teilstrom als Bypassstrom angesehen werden, der an der Bestrahlungseinrichtung vorbei oder herumgeführt wird.

Im Hinblick auf Vorteile und bevorzugte Ausführungsformen darf auf die vorangegangenen Ausführungen zu der Reinhaltungsvorrichtung verwiesen werden, die in gleicher Weise auch für das erfindungsgemäße Verfahren gelten.

Mit dem erfindungsgemäßen Verfahren wird somit eine effiziente Inaktivierung von in dem Medium befindlichen Mikroorganismen bereitgestellt und somit eine effiziente Reinigung des Mediums, insbesondere von Luft, ermöglicht. Erfindungsgemäß ist mit dem Verfahren die Möglichkeit geschaffen worden, dass eine Luftdesinfektion insbesondere nur für einen Teilstrom des gesamten Mediumstroms erfolgt.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, auch wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt:

Fig. 1      eine schematische perspektivische Darstellung einer erfindungsgemäßen Reinhaltungsvorrichtung,

Fig. 2      eine schematische Seitenansicht einer weiteren Ausführungsform einer erfindungsgemäßen Reinhaltungsvorrichtung,

Fig. 3      eine schematische Prinzipskizze einer weiteren Ausführungsform einer erfindungsgemäßen Reinhaltungsvorrichtung,

Fig. 4      eine schematische Prinzipskizze einer weiteren Ausführungsform einer erfindungsgemäßen Reinhaltungsvorrichtung,

Fig. 5      eine schematische Prinzipskizze einer weiteren Ausführungsform einer erfindungsgemäßen Reinhaltungsvorrichtung,

Fig. 6      eine schematische Prinzipskizze einer weiteren Ausführungsform einer erfindungsgemäßen Reinhaltungsvorrichtung,

Fig. 7      eine schematische Prinzipskizze einer weiteren Ausführungsform einer erfindungsgemäßen Reinhaltungsvorrichtung,

Fig. 8      eine schematische Prinzipskizze einer weiteren Ausführungsform einer erfindungsgemäßen Reinhaltungsvorrichtung,

Fig. 9      eine schematische Prinzipskizze einer weiteren Ausführungsform einer erfindungsgemäßen Reinhaltungsvorrichtung,

Fig. 10      eine schematische perspektivische Darstellung einer weiteren Ausführungsform einer erfindungsgemäßen Reinhaltungsvorrichtung,

Fig. 11      eine schematische perspektivische Darstellung einer weiteren Ausführungsform einer erfindungsgemäßen Reinhaltungsvorrichtung,

Fig. 12    eine schematische perspektivische Darstellung einer weiteren Ausführungsform einer erfindungsgemäßen Reinhaltungsvorrichtung,

Fig. 13    eine schematische perspektivische Darstellung einer weiteren Ausführungsform einer erfindungsgemäßen Reinhaltungsvorrichtung und

Fig. 14    eine schematische perspektivische Darstellung einer weiteren Ausführungsform einer erfindungsgemäßen Reinhaltungsvorrichtung,

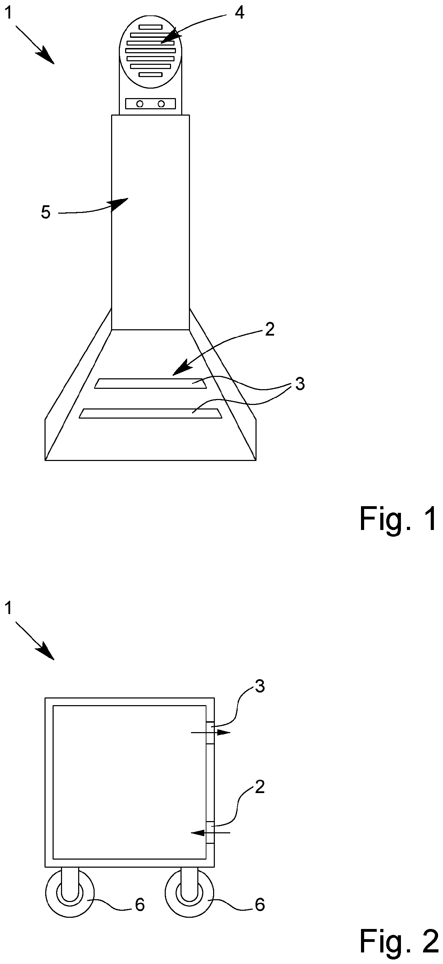

Fig. 1 zeigt eine Reinhaltungsvorrichtung 1, die zur Inaktivierung bzw. Abtötung von Mikroorganismen vorgesehen ist, wobei sich die Mikroorganismen in einem die Reinhaltungsvorrichtung 1 durchströmenden Medium befinden. Das die Reinhaltungsvorrichtung 1 durchströmende Medium kann Wasser oder Luft bzw. ein Gasgemisch und/oder ein Dampfgemisch sein. Die zu inaktivierenden Mikroorganismen können insbesondere Bakterien, Keime, Schimmel und/oder Viren sein.

In Fig. 1 ist dargestellt, dass die Reinhaltungsvorrichtung 1 als insbesondere mobiles Standgerät ausgebildet ist, wobei das Medium Luft ist und über einen Einlass 2, der eine Mehrzahl von Einlassöffnungen 3 aufweisen kann, in die Reinhaltungsvorrichtung 1 eingesogen wird. Das Medium verlässt die Reinhaltungsvorrichtung 1 über einen Auslass 4, der ebenfalls eine Mehrzahl von Auslassöffnungen 12 aufweisen kann. Sowohl der Einlass 2 als auch der Auslass 4 sind in bzw. an einem ein- oder mehrstückigen Gehäuse 5 angeordnet. Der Einlass 2 oder der Auslass 4 können jeweils an wenigstens einer Wandung des Gehäuses 5 oder aber verteilt an einer Mehrzahl von Wandungen angeordnet sein.

Fig. 2 zeigt eine mobile Reinhaltungsvorrichtung 1, die beispielsweise über Räder 6 bewegt bzw. verfahren werden kann. Das Medium wird über den Einlass 2 in die Reinhaltungsvorrichtung 1 eingesogen und tritt aus der Reinhaltungsvorrichtung 1 über den Auslass 4 aus. Der Strömungsverlauf ist schematisch über Pfeile dargestellt.

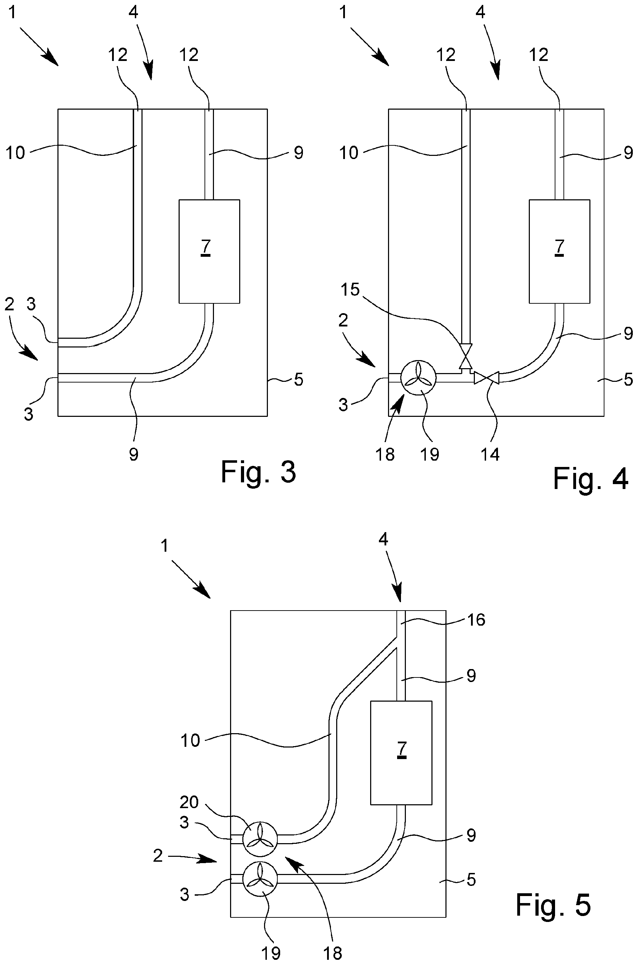

In Fig. 3 ist dargestellt, dass die Reinhaltungsvorrichtung 1 eine Bestrahlungseinrichtung 7 aufweist. Die Bestrahlungseinrichtung 7 ist zur UV-Bestrahlung, insbesondere UV-C-Bestrahlung, des Mediums vorgesehen.

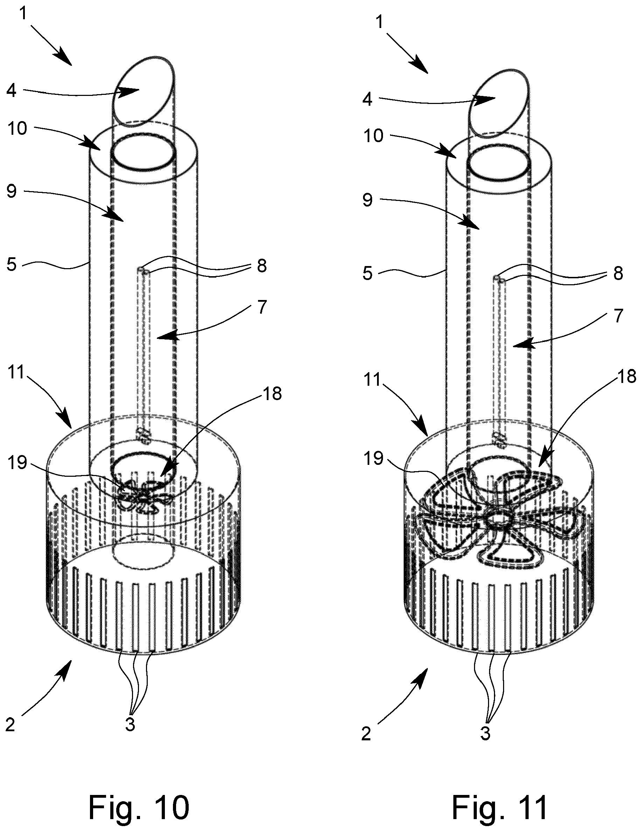

Die Bestrahlungseinrichtung 7 kann wenigstens eine Strahlungsquelle 8 aufweisen, wie dies beispielsweise in Fig. 11 dargestellt ist, wobei in Fig. 11 zwei Strahlungsquellen 8 gezeigt sind.

Die Strahlungsquelle 8 kann UV-Strahlung in einem Wellenlängenbereich von wenigstens 240 nm bis 300 nm, bevorzugt in einem Wellenlängenbereich von 254 nm +/- 10 % und/oder von 278 nm +/- 10 % emittieren. Grundsätzlich kann vorgesehen sein, dass in der Bestrahlungseinrichtung 7 eine Mehrzahl von Strahlungsquellen 8 angeordnet ist, insbesondere zwischen 2 bis 5. Die Strahlungsquelle 8 kann dabei einen Durchmesser von wenigstens 1 cm aufweisen. Als Strahlungsquelle 8 können auch eine Mehrzahl von Leuchtmitteln, vorzugsweise LEDs, in weiteren Ausführungsformen dienen. In der Fig. 11 ist eine stabförmige Form der Strahlungsquelle 8 dargestellt, die insbesondere eine Länge zwischen 10 cm bis 20 cm aufweist. Die in Fig. 1 dargestellte Reinhaltungsvorrichtung 1 ist insbesondere in dem Gehäuse 5 angeordnet, wobei das Gehäuse 5 eine Länge bzw. Höhe zwischen 80 bis 150 cm aufweisen kann.

Die Fig. 3 bis 9 zeigen Prinzipskizzen der Funktionsweisen von unterschiedlichen Ausführungsformen einer erfindungsgemäßen Reinhaltungsvorrichtung 1.

In Fig. 3 ist dargestellt, dass ein Leitungsmittel 9 vorgesehen ist, wobei das Leitungsmittel 9 die Bestrahlungseinrichtung 7 strömungsmäßig mit dem Einlass 3 verbindet. Das Leitungsmittel 9 dient zur Zuführung eines Teilstroms des Mediums zu der Bestrahlungseinrichtung 7.

Die Bestrahlungseinrichtung 7 kann als separates Bauteil ausgebildet sein.

Alternativ ist es möglich, dass die Bestrahlungseinrichtung 7 durch einen Abschnitt des Leitungsmittels 9 gebildet wird, in dem die wenigstens eine Strahlungsquelle 8 angeordnet ist. Dies ist schematisch in den Fig. 10 bis 14 dargestellt.

Insbesondere ist das Leitungsmittel 9 innerhalb des Gehäuses 5 angeordnet.

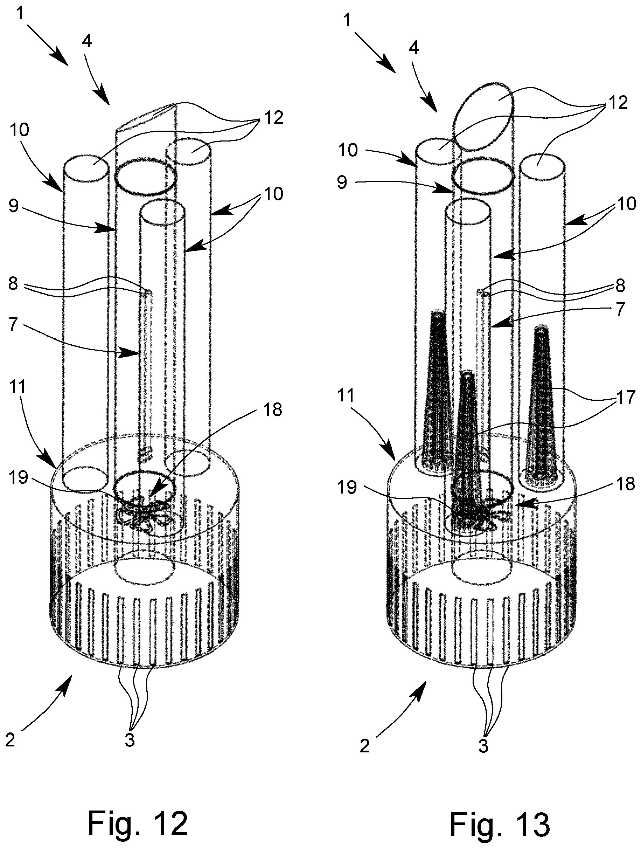

Zusätzlich zu dem Leitungsmittel 9 weist die in Fig. 3 dargestellte Reinhaltungsvorrichtung 1 ein weiteres Leitungsmittel 10 auf. Bei den in den Fig. 3 bis 9 dargestellten Ausführungsbeispielen ist nur ein weiteres Leitungsmittel vorgesehen. Grundsätzlich kann auch eine Mehrzahl von weiteren Leitungsmitteln in der Reinhaltungsvorrichtung 1 eingesetzt werden, wie beispielsweise schematisch in der Fig. 12 dargestellt ist. So können insbesondere zwischen 2 bis 5 weitere Leitungsmittel 10 vorgesehen sein. Die weiteren Leitungsmittel 10 dienen zur Führung eines weiteren Teilstroms des Mediums. Der weitere Teilstrom wird nicht durch die Bestrahlungseinrichtung 7 geführt bzw. der Strömungspfad des weiteren Teilstroms verläuft nicht durch die Bestrahlungseinrichtung 7. Das weitere Leitungsmittel 10 mündet auch nicht in die Bestrahlungseinrichtung 7.

In Fig. 3 ist dargestellt, dass das weitere Leitungsmittel 10 an der Bestrahlungseinrichtung 7 vorbeigeführt ist und ferner von der Bestrahlungseinrichtung 7 räumlich getrennt ist bzw. nicht unmittelbar mit der Bestrahlungseinrichtung 7 verbunden ist. Auch ist der in dem weiteren Leitungsmittel 10 geführte weitere Teilstrom strömungsmäßig getrennt von dem in dem Leitungsmittel 9 geführten Teilstrom.

In Fig. 4 ist dargestellt, dass das weitere Leitungsmittel 10 letztlich als Bypass ausgebildet ist, der an der Bestrahlungseinrichtung 7 vorbeigeführt wird.

Bei den in den Fig. 10 und 11 dargestellten Ausführungsbeispielen ist vorgesehen, dass das Leitungsmittel 9 von dem weiteren Leitungsmittel 10 umgeben bzw. umschlossen ist. Letztlich ist aber die durch das weitere Leitungsmittel 10 geführte weitere Teilströmung unabhängig von dem in dem Leitungsmittel 9 geführten Teilstrom, der der Bestrahlungseinrichtung 7 zugeführt wird. Auch bei der in Fig. 11 gezeigten Ausführungsform sind wenigstens zwei Teilströme vorgesehen, die strömungsmäßig voneinander getrennt sind.

Fig. 5 zeigt, dass diese Teilströme vor Zuführung zu dem Auslass 4 wieder miteinander vereinigt werden können. Zumindest im Bereich (in Strömungsrichtung gesehen) der Bestrahlungseinrichtung 7 ist der in dem Leitungsmittel 9 geführte Teilstrom strömungstechnisch bzw. -mäßig unabhängig bzw. getrennt von dem in dem weiteren Leitungsmittel 10 geführten weiteren Teilstrom.

Demnach wird bei dem in Fig. 3 dargestellten Ausführungsbeispiel der Bestrahlungseinrichtung 7 nur ein Teilstrom des die Reinhaltungsvorrichtung 1 durchströmenden Stromes des Mediums über das Leitungsmittel 9 zugeführt. Der restliche Strom des die Reinhaltungsvorrichtung 1 durchströmenden Stromes des Mediums wird durch das wenigstens eine weitere Leitungsmittel 10 geführt.

Zudem zeigt Fig. 3, dass das weitere Leitungsmittel 10 strömungsmäßig von dem Leitungsmittel 9 getrennt ist, wobei das weitere Leitungsmittel 10 den Einlass 2 strömungsmäßig mit dem Auslass 4 verbindet. Bei dem in den Fig. 3 und 4 dargestellten Ausführungsbeispielen ist vorgesehen, dass das Leitungsmittel 9 den Teilstrom nach Austritt aus der Bestrahlungseinrichtung 7 zu dem Auslass 4 führt. Das Leitungsmittel 9 geht zumindest bis zur Zuführung zu der Bestrahlungseinrichtung 7.

Erfindungsgemäß kann beispielsweise der durch die Bestrahlungseinrichtung 7 geführte Teilstrom einen Volumenstrom von 450 m$^3$/h +/- 10 % aufweisen, der mit einem weiteren Teilstrom von etwa 200 m$^3$/h +/- 20 % gemischt werden kann. Beim ersten Durchgang durch die Reinhaltungsvorrichtung 1 kann insbesondere eine Abscheidung der schädlichen Mikroorganismen von wenigstens 95 %, bevorzugt wenigstens 98 % +/- 2 % erreicht werden.

In Fig. 12 ist dargestellt, dass das Leitungsmittel 9 und das wenigstens eine weitere Leitungsmittel 10 als geschlossener Strömung-Führungskanal ausgebildet sind. Bei dem in dem in Fig. 12 dargestellten Ausführungsbeispiel ist als Leitungsmittel 9 und als weiteres Leitungsmittel 10 insbesondere ein Rohr vorgesehen. Die Bestrahlungseinrichtung 7 kann mit ihrer Strahlungsquelle 8 auch innerhalb des Leitungsmittels 9 angeordnet sein. Das die Bestrahlungseinrichtung 7 durchströmende Medium wird insbesondere über eine UV-Behandlung desinfiziert.

Bei dem in der Fig. 10 dargestellten Ausführungsbeispiel ist vorgesehen, dass der Einlass 2 im Bereich eines Sockels 11 der Reinhaltungsvorrichtung 1 angeordnet ist.

Der Sockel 11 kann letztlich als Standfuß ausgebildet sein. Umfangsmäßig sind um den Sockel 11 eine Mehrzahl von Einlassöffnungen 3 vorgesehen, die zueinander beabstandet und in dem in Fig. 10 dargestellten Ausführungsbeispiel insbesondere schlitzförmig ausgebildet sind. Bei dem in Fig. 10 dargestellten Ausführungsbeispiel sind die Einlassöffnungen 3 sowohl für das Leitungsmittel 9 als auch für das weitere Leitungsmittel 10 vorgesehen bzw. diesen zugeordnet.

Bei dem in Fig. 3 dargestellten Ausführungsbeispiel ist vorgesehen, dass je eine Einlassöffnung 3 für das Leitungsmittel 9 und das weitere Leitungsmittel 10 vorgesehen sind. Nicht dargestellt ist, dass auch eine Mehrzahl von Einlassöffnungen 3 für das Leitungsmittel 9 und das weitere Leitungsmittel 10 vorgesehen sein können. Insbesondere kann die dem Leitungsmittel 9 zugeordnete Einlassöffnung 3 nicht gleichzeitig dem weiteren Leitungsmittel 10 zugeordnet sein.

In Fig. 4 ist dargestellt, dass der Auslass 4 eine Mehrzahl von Auslassöffnungen 12 aufweist. Bei dem in Fig. 4 dargestellten Ausführungsbeispiel ist dem Leitungsmittel 9 eine Auslassöffnung 12 und dem weiteren Leitungsmittel 10 eine weitere Auslassöffnung 12 zugeordnet. Die Auslassöffnungen 12 können derart angeordnet und ausgebildet sein, dass sie je nur einem Leitungsmittel 9, 10 zugeordnet sind. Die Auslassöffnungen 12 und/oder die Einlassöffnungen 3 können grundsätzlich an derselben Wandung des Gehäuses 5 oder an unterschiedlichen Wandungen angeordnet sein.

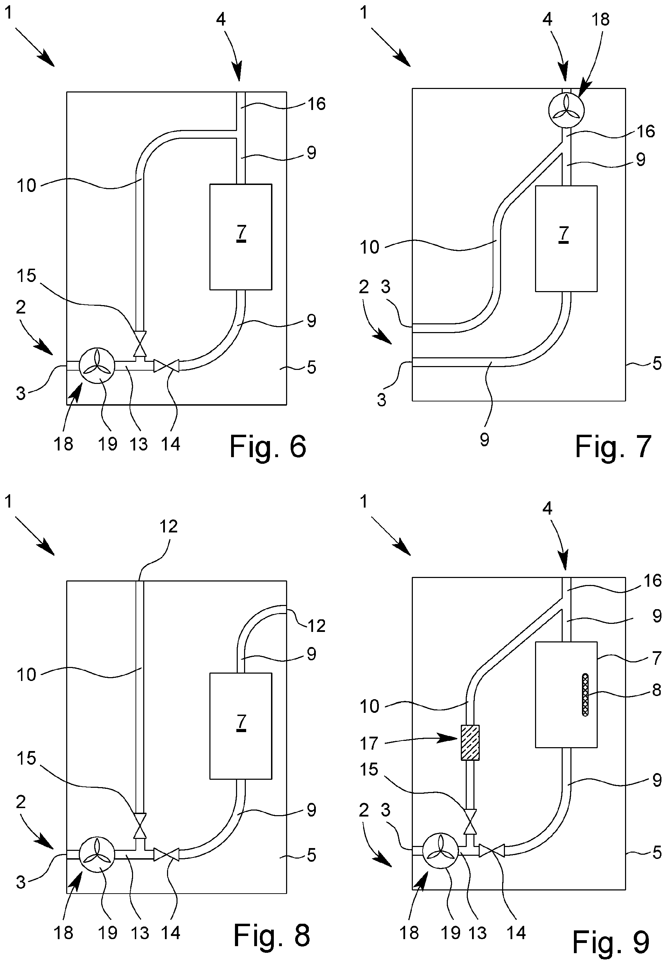

In Fig. 8 ist dargestellt, dass die Auslassöffnung 12 des Leitungsmittels 9 an einer Seitenwandung angeordnet ist, wohingegen die Auslassöffnung 12 des weiteren Leitungsmittels 10 an einer Oberseite des Gehäuses 5 angeordnet ist.

In Fig. 8 ist dargestellt, dass ein Einlassmittel 13 vorgesehen ist. Das Einlassmittel 13 kann insbesondere dem Einlass 2 und wenigstens einer Einlassöffnung 3 zugeordnet sein. Bei dem in Fig. 8 dargestellten Ausführungsbeispiel ist das Einlassmittel 13 letztlich als Einlasskanal ausgebildet, von dem aus das Leitungsmittel 9 und das weitere Leitungsmittel 10 abgezweigt sein können. In Fig. 8 ist dargestellt, dass zur Steuerung und zur Zuführung des jeweiligen Teilstroms Stellmittel 14, 15 vorgesehen sind. Die Stellmittel 14, 15 können als Stellklappen oder, wie in Fig. 8 schematisch dargestellt ist, als Ventile ausgebildet sein. Die Stellmittel 14, 15 können zur Steuerung des Volumenstroms des in das Leitungsmittel 9 geführten Teilstroms und/oder des in das weitere Leitungsmittel 10 geführten weiteren Teilstroms vorgesehen sein.

In Fig. 5 ist dargestellt, dass ein Auslassmittel 16 vorgesehen ist. Das Auslassmittel 16 mündet in dem Auslass 4 und insbesondere in wenigstens einer Auslassöffnung 12. Das Auslassmittel 16 kann als Kanal ausgebildet sein. In dem in Fig. 5 dargestellten Ausführungsbeispiel ist vorgesehen, dass das Leitungsmittel 9 und das weitere Leitungsmittel 10 in das Auslassmittel 16 münden. Insbesondere kann in dem Auslassmittel 16 eine Mischung und/oder Zusammenführung des Teilstroms und des wenigstens einen weiteren Teilstroms vorgesehen sein. Das Auslassmittel 16 ist strömungsmäßig - in Strömungsrichtung gesehen - hinter der Bestrahlungseinrichtung 7 angeordnet.

Nicht dargestellt ist, dass die Reinhaltungsvorrichtung 1 derart ausgebildet sein kann, dass der aus der Bestrahlungseinrichtung 7 austretende Teilstrom eine um wenigstens 20 %, bevorzugt wenigstens 30 %, höhere Geschwindigkeit als der weitere Teilstrom aufweist. Diese erhöhte Geschwindigkeit kann insbesondere dazu verwendet werden, dass eine ausreichend hohe Geschwindigkeit des Volumenstroms des weiteren Teilstroms in dem weiteren Leitungsmittel 10 erreicht wird. Beispielsweise zeigt Fig. 12, dass die in den weiteren Leitungsmitteln 10 vorhandene Strömung letztlich durch die Strömung des durch die Bestrahlungseinrichtung 7 geführten Teilstroms ermöglicht werden kann.

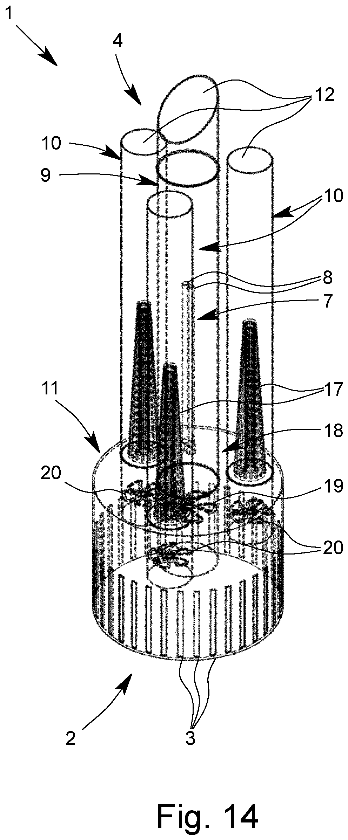

In Fig. 9 ist schematisch dargestellt, dass in dem weiteren Leitungsmittel 10 eine Filtereinrichtung 17 angeordnet ist. Filtereinrichtungen 17 sind ferner auch bei den in den Fig. 13 und 14 dargestellten Ausführungsbeispielen vorgesehen. Als Filtereinrichtung 17 kann beispielsweise ein Hepa-Filter vorgesehen sein. Die Filtereinrichtung 17 kann derart ausgebildet sein, dass Partikel mit einem Durchmesser von größer als 0,5 μm aus dem jeweils durch die Filtereinrichtung 17 geführten Teilstrom gefiltert werden.

Nicht dargestellt ist, dass vor dem Einlass 2 und/oder in dem Leitungsmittel 9 ein Vorfilter angeordnet ist, insbesondere wobei der Vorfilter derart ausgebildet ist, dass Partikel mit einem Durchmesser von größer als 0,5 μm aus dem Mediumstrom gefiltert werden.

In Fig. 4 bis 9 ist schematisch dargestellt, dass eine Gebläseeinrichtung 18 eingesetzt wird. Die Gebläseeinrichtung 18 kann wenigstens einen Ventilator 19 aufweisen. In dem in Fig. 5 dargestellten Ausführungsbeispiel ist vorgesehen, dass die Gebläseeinrichtung 18 neben dem Ventilator 19 einen weiteren Ventilator 20 aufweist. Der Ventilator 19 kann zum Ansaugen und zum Abblasen des Teilstroms, der durch die Bestrahlungseinrichtung 7 geführt wird, ausgebildet sein.

Zudem kann über den Ventilator auch der weitere Teilstrom erzeugt werden, der vom Einlass 2 zum Auslass 4 geführt wird. Dies ist beispielsweise bei dem in Fig. 11 dargestellten Ausführungsbeispiel vorgesehen.

Fig. 5 zeigt, dass die Gebläseeinrichtung 18 zur Erzeugung des weiteren Teilstroms vom Einlass 2 zu dem Auslass 4 einen weiteren Ventilator 20 aufweist. Weitere Ventilatoren 20 sind auch in dem in Fig. 14 dargestellten Ausführungsbeispiel vorgesehen.

Bei den Ausführungsbeispielen gemäß den Fig. 10, 12 und 13 weist die Gebläseeinrichtung 18 einen Ventilator 19 auf, der für das Leitungsmittel 9 und letztlich zur Erzeugung des der Bestrahlungseinrichtung 7 zugeführten Teilstroms vorgesehen ist. Durch den Venturi-Effekt bzw. durch strömungstechnische Effekte kann jedoch durch die erhöhte Geschwindigkeit des aus der Bestrahlungseinrichtung 7 austretenden Teilstroms auch ein ausreichend hoher Volumenstrom des wenigstens einen weiteren Teilstroms sichergestellt werden.

In Fig. 7 ist dargestellt, dass die Gebläseeinrichtung 18 mit einem Ventilator 19 - in Strömungsrichtung gesehen - hinter der Bestrahlungseinrichtung 7 angeordnet ist.

Die weiteren Ausführungsbeispiele verdeutlichen jedoch, dass die Gebläseeinrichtung 18 bevorzugt - in Strömungsrichtung gesehen - vor der Bestrahlungseinrichtung 7 angeordnet ist und vorzugsweise zumindest dem Leitungsmittel 9 zugeordnet ist.

Die Stellmittel 14, 15 können zudem der Gebläseeinrichtung 18 zugeordnet bzw. zu der Gebläseeinrichtung 18 gekoppelt sein.

Nicht dargestellt ist, dass die Bestrahlungseinrichtung 7 zur Behandlung eines Volumenstroms des Teilstroms von wenigstens 200 m³/h ausgebildet ist. Zudem kann die Reinhaltungsvorrichtung 1 derart ausgebildet sein, dass der Volumenstrom des durch das Leitungsmittel 9 geführten Teilstroms an dem Gesamtvolumenstrom der Reinhaltungsvorrichtung 1 durchströmenden Mediumstroms ein Anteil von wenigstens 10 %, bevorzugt zwischen 30 bis 50 %, aufweist.

Bei den Fig. 12 bis 14 ist dargestellt, dass die weiteren Leitungsmittel 10 zumindest im Wesentlichen parallel zu dem Leitungsmittel 9 angeordnet sind und vorzugsweise zu dem Leitungsmittel 9 beabstandet sind. Dabei kann das Leitungsmittel 9 von den weiteren Leitungsmitteln 10 umgeben sein bzw. von diesen eingefasst sein. Bei den Ausführungsbeispielen gemäß den Fig. 10 und 11 ist dargestellt, dass das Leitungsmittel 9 letztlich innerhalb des weiteren Leitungsmittels 10 derart angeordnet ist, dass der Strömungsweg bzw. Strömungspfad des durch die Bestrahlungseinrichtung 7 geführten Teilstroms insbesondere unabhängig von dem Strömungsweg bzw. Strömungspfad der weiteren Teilströmung ist. Letztlich ist das weitere Leitungsmittel 10 bei den Ausführungsbeispielen gemäß den Fig. 10 und 11 der Zwischenraum zwischen den dargestellten Kanälen bzw. Rohren.

Nicht dargestellt ist, dass ist, dass eine Steuerungseinrichtung zur Steuerung, Zuschaltung, Abschaltung und/oder Regelung des Volumenstroms, des Teilstroms und/oder des wenigstens einen weiteren Teilstroms vorgesehen ist. Insbesondere kann die nicht näher dargestellte Steuerungseinrichtung zur Steuerung und/oder Regelung des Stellmittels 14 und/oder des weiteren Stellmittels 15 ausgebildet sein.

Insbesondere ist das Leitungsmittel 9 und das wenigstens eine weitere Leitungsmittel 10 zumindest im Wesentlichen vollständig im Gehäuse 5 angeordnet.

**Bezugszeichenliste:**

1    Reinhaltungsvorrichtung

2    Einlass
3    Einlassöffnung
4    Auslass
5    Gehäuse
6    Räder
7    Bestrahlungseinrichtung
8    Strahlungsquelle
9    Leitungsmittel
10   weiteres Leitungsmittel
11   Sockel
12   Auslassöffnung
13   Einlassmittel
14   Stellmittel
15   weiteres Stellmittel
16   Auslassmittel
17   Filtereinrichtung
18   Gebläseeinrichtung
19   Ventilator
20   weiterer Ventilator

**Patentansprüche**

1.   Reinhaltungsvorrichtung (1) zur Inaktivierung von in einem die Reinhaltungsvorrichtung (1) durchströmenden Medium, nämlich Luft, befindlichen Mikroorganismen, wie Bakterien, Keime, Schimmel und/oder Viren, mit einer Bestrahlungseinrichtung (7) zur UV-Bestrahlung, insbesondere UV-C-Bestrahlung, des Mediums, wobei in der Bestrahlungseinrichtung (7) mindestens eine Strahlungsquelle (8) zur Bestrahlung des die Bestrahlungseinrichtung (7) durchströmenden Mediums bzw. des Teilstroms vorgesehen ist, wobei mindestens eine Strahlungsquelle (8) UV-Strahlung in einem Wellenlängenbereich von 250 bis 285 nm emittiert,

wobei ein mit der Bestrahlungseinrichtung (7) strömungsmäßig verbundenes Leitungsmittel (9) zur Zuführung eines Teilstroms des die Reinhaltungsvorrichtung (1) durchströmenden Stromes des Mediums zu der Bestrahlungseinrichtung (7) vorgesehen ist,
wobei wenigstens ein weiteres Leitungsmittel (10) zur Führung eines weiteren Teilstroms vorgesehen ist und wobei das weitere Leitungsmittel (10) nicht in die Bestrahlungseinrichtung (7) mündet und an der Bestrahlungseinrichtung (7) vorbeigeführt ist und von der Bestrahlungseinrichtung (7) räumlich und strömungsmäßig getrennt ist,
wobei eine Gebläseeinrichtung (18) zum Ansaugen und/oder Ausblasen des Mediums vorgesehen ist,
wobei die Reinhaltungsvorrichtung (1) derart ausgebildet ist, dass der aus der Bestrahlungseinrichtung (7) austretende Teilstrom eine höhere, bevorzugt um wenigstens 20 %, weiter bevorzugt um wenigstens 40 %, vorzugsweise um wenigstens 50 %, Geschwindigkeit als der wenigstens eine weitere Teilstrom aufweist,
wobei die Gebläseeinrichtung (19) ein Stellmittel (14), insbesondere ein Ventil und/oder eine Stellklappe, zur Steuerung des Volumenstroms des in das Leitungsmittel (9) geführten Teilstroms und wenigstens ein weiteres Stellmittel (15), insbesondere ein Ventil und/oder eine Stellklappe, zur Steuerung des Volumenstroms des wenigstens einen in das wenigstens eine weitere Leitungsmittel (10) geführten weiteren Teilstroms aufweist,
wobei eine Steuerungseinrichtung zur Steuerung, Zuschaltung, Abschaltung und/oder Regelung des Volumenstromes des Teilstroms und/oder des wenigstens einen weiteren Teilstroms vorgesehen ist.

2.   Reinhaltungsvorrichtung nach Anspruch 1, **dadurch gekennzeichnet, dass** ein einen Einlass (2) und einen Auslass (4) für das Medium aufweisendes Gehäuse (5) vorgesehen ist, insbesondere wobei das Gehäuse (5) von dem durch die Reinhaltungsvorrichtung (1) geführten Strom des Mediums durchströmbar ist und/oder insbesondere wobei die Bestrahlungseinrichtung (7) in dem Gehäuse (5) angeordnet ist.

3.   Reinhaltungsvorrichtung nach Anspruch 1 oder 2, **dadurch gekennzeichnet, dass** das Leitungsmittel (9) den Einlass (2) mit der Bestrahlungseinrichtung (7) strömungsmäßig verbindet und/oder dass das weitere Leitungsmittel (10) strömungsmäßig mit dem Einlass (2) verbunden ist, insbesondere wobei das weitere Leitungsmittel (10) den Einlass (2) strömungsmäßig mit dem Auslass (4) verbindet und/oder insbesondere wobei das Leitungsmittel (9) den Einlass (2) strömungsmäßig mit dem Auslass (4) verbindet.

4.   Reinhaltungsvorrichtung nach einem der vorhergehenden Ansprüche, **dadurch gekennzeichnet, dass** eine Mehrzahl von weiteren Leitungsmitteln (10) zur Führung einer Mehrzahl von weiteren Teilströmen vorgesehen ist.

5.   Reinhaltungsvorrichtung nach einem der vorhergehenden Ansprüche, **dadurch gekennzeichnet, dass** der Einlass (2) eine Mehrzahl an Einlassöffnungen (3) aufweist und/oder

dass der Auslass (4) eine Mehrzahl von Auslassöffnungen (12) aufweist,
insbesondere wobei dem Leitungsmittel (9) wenigstens eine Einlassöffnung (3) und/oder dem wenigstens einen weiteren Leitungsmittel (10) wenigstens eine weitere Einlassöffnung (3) zugeordnet ist und/oder

insbesondere wobei dem Leitungsmittel (9) wenigstens eine Auslassöffnung (12) und/oder dem wenigstens einen weiteren Leitungsmittel (10) wenigstens eine weitere Auslassöffnung (12) zugeordnet ist.

6. Reinhaltungsvorrichtung nach einem der vorhergehenden Ansprüche, **dadurch gekennzeichnet, dass** ein strömungsmäßig mit dem Auslass (4) verbundenes Auslassmittel (16) vorgesehen ist, wobei das Leitungsmittel (9) und/oder der aus der Bestrahlungseinrichtung (7) austretende Teilstrom und das wenigstens eine weitere Leitungsmittel (10) in das Auslassmittel (16) münden, vorzugsweise zur Mischung des aus der Bestrahlungseinrichtung (7) austretenden Teilstroms und des wenigstens einen weiteren Teilstroms.

7. Reinhaltungsvorrichtung nach einem der vorhergehenden Ansprüche, **dadurch gekennzeichnet, dass** in dem wenigstens einen weiteren Leitungsmittel (10) eine Filtereinrichtung (17) angeordnet ist, insbesondere wobei die Filtereinrichtung (17) derart ausgebildet ist, dass Partikel mit einem Durchmesser größer 1 μm, bevorzugt größer 0,5 μm, aus dem Mediumstrom gefiltert werden.

8. Reinhaltungsvorrichtung nach einem der vorhergehenden Ansprüche, **dadurch gekennzeichnet, dass** vor dem Einlass (2) und/oder im Bereich des Einlasses (2) und/oder in dem Leitungsmittel (9) ein Vorfilter angeordnet ist, insbesondere wobei der Vorfilter derart ausgebildet ist, dass Partikel mit einem Durchmesser größer 1 μm, bevorzugt größer 0,5 μm, aus dem Mediumstrom gefiltert werden.

9. Reinhaltungsvorrichtung nach einem der vorhergehenden Ansprüche, **dadurch gekennzeichnet, dass** die Gebläseeinrichtung (18) einen Ventilator (19) zum Erzeugen des Teilstroms in dem Leitungsmittel (9) von dem Einlass (2) zu der Bestrahlungseinrichtung (7), bevorzugt zu dem Auslass (4), aufweist und/oder dass die Gebläseeinrichtung (18) wenigstens einen weiteren Ventilator (20) zum Erzeugen des wenigstens einen weiteren Teilstroms in dem wenigstens einen weiteren Leitungsmittel (10) von dem Einlass (2) zu dem Auslass (4) aufweist.

10. Reinhaltungsvorrichtung nach einem der vorhergehenden Ansprüche, **dadurch gekennzeichnet, dass** die Bestrahlungseinrichtung (7) zur Behandlung eines Volumenstromes des Teilstroms von wenigstens 100 m³/h, bevorzugt von wenigstens 200 m³/h, weiter bevorzugt zwischen 300 bis 600 m³/h, ausgebildet ist.

11. Reinhaltungsvorrichtung nach einem der vorhergehenden Ansprüche, **dadurch gekennzeichnet, dass** die Reinhaltungsvorrichtung (1) derart ausgebildet ist, dass der Volumenstrom des durch das Leitungsmittel (9) geführten Teilstroms an dem gesamten Volumenstrom des die Reinhaltungsvorrichtung (1) durchströmenden Mediumstroms einen Anteil von wenigstens 10 %, bevorzugt zwischen 20 % bis 80 %, weiter bevorzugt zwischen 30 % bis 50 %, aufweist.

12. Reinhaltungsvorrichtung nach einem der vorhergehenden Ansprüche, **dadurch gekennzeichnet, dass** die Steuerungseinrichtung zur Steuerung und/oder Regelung des Stellmittels (14) und/oder des wenigstens einen weiteren Stellmittels (15) ausgebildet ist.

## Claims

1. Purification device (1) for inactivating microorganisms, such as bacteria, germs, mold and/or viruses, present in a medium flowing through the purification device (1), namely air, with an irradiation device (7) for UV irradiation, in particular UV-C irradiation, of the medium, wherein at least one radiation source (8) is provided in the irradiation device (7) for irradiating the medium and/or the partial flow flowing through the irradiation device (7), wherein at least one radiation source (8) emits UV radiation in a wavelength range of 250 to 285 nm,

wherein a guiding means (9) flow-connected to the irradiation device (7) is provided for supplying a partial flow of the flow of the medium flowing through the purification device (1) to the irradiation device (7),

wherein at least one further guiding means (10) is provided for guiding a further partial flow and wherein the further guiding means (10) does not open into the irradiation device (7) and is guided past the irradiation device (7) and is spatially and flow-wise separated from the irradiation device (7),

wherein a blower device (18) is provided for sucking in and/or blowing out the medium,

wherein the purification device (1) is designed such that the partial flow emerging from the irradiation device (7) has a higher velocity, preferably by at least 20 %, more preferably by at least 40 %, preferably by at least 50 %, than the at least one further partial flow,

wherein the blower device (19) has an actuating means (14), in particular a valve and/or a butterfly valve, for controlling the volumetric flow of the partial flow guided into the guiding means (9) and at least one further actuating means (15), in particular a valve and/or a butterfly valve, for controlling the volumetric flow of the at least one further partial flow guided into the at least one further guiding means (10),

wherein a control device is provided for controlling, connecting, disconnecting and/or regulating the volumetric flow of the partial flow and/or the at least one further partial flow.

2. Purification device according to claim 1, **characterized in that** a housing (5) having an inlet (2) and an outlet (4) for the medium is provided, in particular wherein the housing (5) can be flowed through by the flow of the medium conducted through the purification device (1) and/or in particular wherein the irradiation device (7) is arranged in the housing (5).

3. Purification device according to claim 1 or 2, **characterized in that** the guiding means(9) fluidically connects the inlet (2) to the irradiation device (7) and/or that the further guiding means (10) is fluidically connected to the inlet (2), in particular wherein the further guiding means (10) fluidically connects the inlet (2) to the outlet (4) and/or in particular wherein the guiding means (9) fluidically connects the inlet (2) to the outlet (4).

4. Purification device according to one of the preceding claims, **characterized in that** a plurality of further guiding means (10) is provided for conducting a plurality of further partial flows.

5. Purification device according to one of the preceding claims, **characterized in that** the inlet (2) comprises a plurality of inlet openings (3) and/or

that the outlet (4) has a plurality of outlet openings (12),
in particular wherein at least one inlet opening (3) is associated with the guiding means(9) and/or at least one further inlet opening (3) is associated with the at least one furtherguiding means (10) and/or
in particular wherein at least one outlet opening (12) is associated with the guiding means(9) and/or at least one further outlet opening (12) is associated with the at least one further guiding means (10).

6. Purification device according to one of the preceding claims, **characterized in that** an outlet means (16) is provided which is flow-connected to the outlet (4), wherein the guiding means (9) and/or the partial flow exiting from the irradiation device (7) and the at least one further guiding means (10) open into the outlet means (16), preferably for mixing the partial flow exiting from the irradiation device (7) and the at least one further partial flow.

7. Purification device according to one of the preceding claims, **characterized in that** a filter device (17) is arranged in the at least one further guiding means (10), in particular wherein the filter device (17) is designed in such a way that particles with a diameter greater than 1 $\mu$m, preferably greater than 0.5 $\mu$m, are filtered out of the medium flow.

8. Purification device according to one of the preceding claims, **characterized in that** a prefilter is arranged upstream of the inlet (2) and/or in the region of the inlet (2) and/or in the guiding means (9), in particular the prefilter being designed in such a way that particles with a diameter greater than 1 $\mu$m, preferably greater than 0.5 $\mu$m, are filtered out of the medium flow.

9. Purification device according to one of the preceding claims, **characterized in that** the blower device (18) comprises a fan (19) for generating the partial flow in the guiding means (9) from the inlet (2) to the irradiation device (7), preferably to the outlet (4), and/or that the blower device (18) comprises at least one further fan (20) for generating the at least one further partial flow in the at least one further guiding means (10) from the inlet (2) to the outlet (4).

10. Purification device according to one of the preceding claims, **characterized in that** the irradiation device (7) is designed to treat a volumetric flow of the partial flow of at least 100 m$^3$/h, preferably of at least 200 m$^3$/h, more preferably between 300 to 600 m$^3$/h.

11. Purification device according to one of the preceding claims, **characterized in that** the purification device (1) is designed in such a way that the volumetric flow of the partial flow guided through the guiding means (9) has a proportion of at least 10%, preferably between 20% and 80%, more preferably between 30% and 50%, of the total volumetric flow of the medium flow flowing through the purification device (1).

12. Purification device according to one of the preceding claims, **characterized in that** the control device is designed for controlling and/or regulating the actuating means (14) and/or the at least one further actuating means (15).

**Revendications**

1. Dispositif de purification (1) pour l'inactivation de micro-organismes, tels que des bactéries, des germes, des moisissures et/ou des virus, se trouvant dans un médium traversant le dispositif de purification (1), à savoir l'air, avec un dispositif d'irradiation (7) pour l'irradiation UV, en particulier l'irradiation UV-C, du médium, au moins une source de rayonnement (8) pour l'irradiation du médium et/ou du courant partiel traversant le dispositif d'irradiation (7) étant prévue dans le dispositif d'irradiation (7), au moins une source de rayonnement (8) émettant un rayonnement UV dans une plage de longueurs d'onde de 250 à 285 nm,

dans lequel un moyen de conduite (9) relié en termes d'écoulement au dispositif d'irradiation (7) est prévu pour amener au dispositif d'irradiation (7) un courant partiel du courant du médium traversant le dispositif de purification (1),

dans lequel au moins un autre moyen de conduite (10) est prévu pour le guidage d'un autre courant partiel et dans lequel l'autre moyen de conduite (10) ne débouche pas dans le dispositif d'irradiation (7) et passe devant le dispositif d'irradiation (7) et est séparé du dispositif d'irradiation (7) dans l'espace et en termes d'écoulement,

un dispositif de soufflage (18) étant prévu pour aspirer et/ou souffler le fluide,

le dispositif de purification (1) étant conçu de telle sorte que le courant partiel sortant du dispositif d'irradiation (7) présente une vitesse plus élevée, de préférence d'au moins 20 %, plus préférablement d'au moins 40 %, de préférence d'au moins 50 %, que le au moins un autre flux partiel,

le dispositif de soufflage (19) présentant un moyen de réglage (14), en particulier une soupape et/ou un clapet de réglage, pour la commande du débit volumique du courant partiel guidé dans le moyen de conduite (9) et au moins un autre moyen de réglage (15), en particulier une soupape et/ou un clapet de réglage, pour la commande du débit volumique d'au moins un autre courant partiel guidé dans l'au moins un autre moyen de conduite (10),

un dispositif de commande étant prévu pour la commande, la mise en circuit, la mise hors circuit et/ou la régulation du débit volumique du courant partiel et/ou du au moins un autre courant partiel.

2. Dispositif de purification selon la revendication 1, **caractérisé en ce qu'**il est prévu un boîtier (5) présentant une entrée (2) et une sortie (4) pour le fluide, en particulier le boîtier (5) pouvant être traversé par le courant de médium passant par le dispositif de purification (1) et/ou en particulier le dispositif d'irradiation (7) étant disposé dans le boîtier (5).

3. Dispositif de purification selon la revendication 1 ou 2, **caractérisé en ce que** le moyen de conduite (9) relie l'entrée (2) au dispositif d'irradiation (7) en termes d'écoulement et/ou **en ce que** l'autre moyen de conduite (10) est relié à l'entrée (2) en termes d'écoulement, en particulier dans lequel l'autre moyen de conduite (10) relie l'entrée (2) à la sortie (4) en termes d'écoulement et/ou en particulier dans lequel le moyen de conduite (9) relie l'entrée (2) à la sortie (4) en termes d'écoulement.

4. Dispositif de purification selon l'une des revendications précédentes, **caractérisé en ce qu'**une pluralité d'autres moyens de conduite (10) est prévue pour conduire une pluralité d'autres courants partiels.

5. Dispositif de purification selon l'une des revendications précédentes, **caractérisé en ce que** l'entrée (2) présente une pluralité d'ouvertures d'entrée (3) et/ou

   **en ce que** la sortie (4) présente une pluralité d'ouvertures de sortie (12), en particulier, au moins une ouverture d'entrée (3) étant associée au moyen de conduite (9) et/ou au moins une autre ouverture d'entrée (3) étant associée à au moins un autre moyen de conduite (10) et/ou en particulier dans lequel au moins une ouverture de sortie (12) est associée au moyen de conduite (9) et/ou au moins une autre ouverture de sortie (12) est associée à au moins un autre moyen de conduite (10).

6. Dispositif de purification selon l'une des revendications précédentes, **caractérisé en ce qu'**il est prévu un moyen de sortie (16) relié en termes d'écoulement à la sortie (4), le moyen de guidage (9) et/ou le courant partiel sortant du dispositif d'irradiation (7) et le au moins un autre moyen de guidage (10) débouchant dans le moyen de sortie (16), de préférence pour mélanger le courant partiel sortant du dispositif d'irradiation (7) et le au moins un autre courant partiel.

7. Dispositif de purification selon l'une des revendications précédentes, **caractérisé en ce qu'**un dispositif de filtration (17) est disposé dans l'au moins un autre moyen de conduite (10), en particulier le dispositif de filtration (17) étant conçu de telle sorte que des particules d'un diamètre supérieur à 1 μm, de préférence supérieur à 0,5 μm, sont filtrées dans le courant de médium.

8. Dispositif de purification selon l'une des revendications précédentes, **caractérisé en ce qu'**un préfiltre est disposé en amont de l'entrée (2) et/ou dans la zone de l'entrée (2) et/ou dans le moyen de conduite (9), en particulier le préfiltre étant conçu de telle sorte que des particules d'un diamètre supérieur à 1 μm, de préférence supérieur à 0,5 μm, sont filtrées dans le flux de fluide.

9.  Dispositif de purification selon l'une des revendications précédentes, **caractérisé en ce que** le dispositif de soufflage (18) comprend un ventilateur (19) pour générer le courant partiel dans le moyen de conduite (9) depuis l'entrée (2) vers le dispositif d'irradiation (7), de préférence vers la sortie (4), et/ou **en ce que** le dispositif de soufflage (18) comprend au moins un autre ventilateur (20) pour générer le au moins un autre courant partiel dans le au moins un autre moyen de conduite (10) depuis l'entrée (2) vers la sortie (4).

10. Dispositif de purification selon l'une des revendications précédentes, **caractérisé en ce que** le dispositif d'irradiation (7) est conçu pour traiter un débit volumique du courant partiel d'au moins 100 m$^3$/h, de préférence d'au moins 200 m$^3$/h, de préférence encore entre 300 et 600 m$^3$/h.

11. Dispositif de purification selon l'une des revendications précédentes, **caractérisé en ce que** le dispositif de purification (1) est conçu de telle sorte que le débit volumique du courant partiel guidé par le moyen de guidage (9) présente, par rapport au débit volumique total du courant de médium traversant le dispositif de purification (1), une part d'au moins 10 %, de préférence comprise entre 20 % et 80 %, plus préférablement entre 30 % et 50 %.

12. Dispositif de purification selon l'une des revendications précédentes, **caractérisé en ce que** le dispositif de commande est conçu pour commander et/ou réguler le moyen de réglage (14) et/ou le au moins un autre moyen de réglage (15).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

**EP 4 082 585 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2020368384 A1
- DE 202009018489 U1
- US 5933702 A
- US 2004041564 A1
- DE 202021100433 U1
- US 2009098014 A1
- DE 3637702 A1